# EUROPEAN PATENT SPECIFICATION

(11) **EP 3 679 115 B1**
(45) Date of publication and mention of the grant of the patent: **02.04.2025**
(21) Application number: 18762112.3
(22) Date of filing: 06.09.2018
(51) Int. Cl.: C11B 9/00, C07C 69/16, C07C 69/28, C07C 43/184, C07C 43/196

(54) **ESTERS AND ETHERS OF 2,2,4,4-TETRAMETHYLCYCLOBUTANE-1,3-DIOL FOR USE AS AROMA CHEMICALS**
ESTER UND ETHER AUS 2,2,4,4-TETRAMETHYLCYCLOBUTAN-1,3-DIOL ZUR VERWENDUNG ALS AROMACHEMIKALIEN
ESTERS ET ÉTHERS DE 2,2,4,4-TÉTRAMÉTHYLCYCLOBUTANE -1,3-DIOL DESTINÉS À ÊTRE UTILISÉS EN TANT QUE PRODUITS CHIMIQUES AROMATIQUES

(30) Priority: 06.09.2017 EP 17189719
(43) Date of publication of application: 15.07.2020
(73) Proprietor: BASF SE, 67056 Ludwigshafen am Rhein (DE)
(72) Inventor: GARLICHS, Florian, 68623 Lampertheim (DE); PINKOS, Rolf, 67056 Ludwigshafen (DE); DUEFERT, Alexander, 67056 Ludwigshafen (DE); BRU ROIG, Miriam, 67056 Ludwigshafen (DE)
(74) Representative: BASF IP Association
(86) International application number: PCT/EP2018/074002
(87) International publication number: WO 2019/048544

(56) References cited:
- US-A- 3 062 852
- US-A- 4 048 120

## Description

The present invention relates to the use of esters and ethers of 2,2,4,4-tetramethylcyclobutane-1,3-diol as aroma chemicals, to aroma chemical compositions comprising at least one ester or ether of 2,2,4,4-tetramethylcyclobutane-1,3-diol and to a method for preparing a fragranced ready-to-use composition, which comprises incorporating at least one ester or ether of 2,2,4,4-tetramethylcyclobutane-1,3-diol into a ready-to-use composition. The present invention further relates to specific ethers and specific esters of 2,2,4,4-tetramethylcyclobutane-1,3-diol and a method for their preparation.

### BACKGROUND OF THE INVENTION

Aroma chemicals, especially fragrances, are of great interest especially in the field of cosmetics and cleaning and laundry compositions. Fragrances of natural origin are mostly expensive, often limited in their available amount and, on account of fluctuations in environmental conditions, are also subject to variations in their content, purity etc. To circumvent these undesirable factors, it is therefore of great interest, to create synthetic substances, which have organoleptic properties that resembles more expensive natural fragrances or which have novel and interesting organoleptic profiles.

Despite a large number of already existing synthetic aroma chemicals (fragrances and flavorings), there is a constant need for new components in order to be able to satisfy the multitude of properties desired for extremely diverse areas of application. These include, firstly, the organoleptic properties, i.e. the compounds should have advantageous odiferous (olfactory) or gustatory properties. Furthermore, aroma chemicals should, however, also have additional positive secondary properties, such as e.g. an efficient preparation method, the possibility of providing better sensory profiles as a result of synergistic effects with other fragrances, a higher stability under certain application conditions, a higher extendability, a better staying power, etc.

However, since even small changes in chemical structure bring about massive changes in the sensory properties such as odor and also taste, the targeted search for substances with certain sensory properties such as a certain odor is extremely difficult. The search for new fragrances and flavorings is therefore in most cases difficult and laborious without knowing whether a substance with the desired odor and/or taste will even actually be found.

DE 1114811 describes a process for the preparation of diesters, where an aliphatic carboxylic acid or benzoic acid is reacted with 2,2,4,4-tetramethylcyclobutane-1,3-diol, 2,4-dimethyl-2,4-diethylcyclobutane-1,3-diol or 2,2,4,4-tetraethylcyclobutane-1,3-diol, and the use of said esters as lubricants, plasticizers, heat transfer oils and hydraulic fluids.

DE 1142695 describes a process for plasticizing cellulose esters using diesters, obtainable from aliphatic carboxylic acid or benzoic acid and 2,2,4,4-tetramethylcyclobutane-1,3-diol, 2,4-dimethyl-2,4-diethylcyclobutane-1,3-diol or 2,2,4,4-tetraethylcyclobutane-1,3-diol, as plasticizer.

US 3,043,791 describes the use of diesters, obtainable from (C₁-C₄)-carboxylic acids and 2,2,4,4-tetramethylcyclobutane-1,3-diol, 2,4-dimethyl-2,4-diethylcyclobutane-1,3-diol or 2,2,4,4-tetraethylcyclobutane-1,3-diol, as plasticizer for polyvinyl chloride and plastic compositions comprising polyvinyl chloride and said diesters.

US 3,062,852 describes diesters, obtainable from aliphatic carboxylic acids and 2,2,4,4-tetra-(C₁-C₄)-alkylcyclobutane-1,3-diols, and the use of said diesters as synthetic lubricants.

US 3,227,764 describes a method for separating a 2,2,4,4-tetra-(C₁-C₄)-alkylcyclobutane-1,3-diol, in particular 2,2,4,4-tetramethylcyclobutane-1,3-diol into its cis and trans isomers, where said diol is reacted with(C₁-C₉)-carboxylic acids to give the corresponding diester, which, due to their different melting temperatures, can then be separated into the individual cis- and trans-isomers and reconverted to the corresponding cis and trans isomers of 2,2,4,4-tetramethylcyclobutane-1,3-diol.

US 4,048,120 relates to the use of compounds for improving, enhancing or modifying the organoleptic properties of perfumes or perfumed products, wherein the compounds derive from 2,2-dimethyl-3-acetylcyclobutylacetic acid.

The use of esters and ethers of 2,2,4,4-tetramethylcyclobutane-1,3-diol as aroma chemicals has so far not been described in the prior art.

### SUMMARY OF THE INVENTION

It is an object of the present invention to provide substances exhibiting plaesant organoleptical properties, which can be advantageously used as aroma chemicals. It was a further object of the present invention to provide substances, which can be used as an aroma chemical in ready-to-use compositions. In particular, odor-intensive substances having a pleasant odor are sought. Furthermore, these aroma chemicals should be combinable with other aroma chemicals, allowing the creation of novel advantageous sensory profiles. In addition, these aroma chemicals should be obtainable from redily available starting materials, allowing their fast and economic manifacturing, and should be free of toxicological concerns.

It was surprisingly found that these and further objects are achieved by esters and ethers of 2,2,4,4-tetramethylcyclobutane-1,3-diol.

Accordingly, a first aspect of the present invention relates to the use of a compound of the general formula (I) 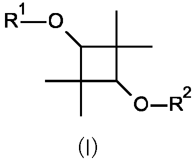 wherein
R¹ is C₁-C₄-alkyl or -(C=O)-R³,
R² is hydrogen, C₁-C₄-alkyl or -(C=O)-R⁴, and
R³ and R⁴, independently of one another, are selected from the group consisting of hydrogen and C₁-C₄-alkyl,
a stereoisomer thereof or a mixture of stereoisomers thereof,
as an aroma chemical.

The use according to the present invention also comprises the use of mixtures of two or more compounds of the general formula (I), which for example differ from each other regarding the radical R¹ and/or regarding the radical R².

The present invention further relates to aroma chemical compositions comprising at least one compound of formula (I), a stereoisomer thereof or a mixture of stereoisomers thereof, as defined above, and at least one further compound (II) selected from the group consisting of aroma chemicals different from compounds (I) and non-aroma chemical carriers;
where the composition is not a mixture comprising as compound of formula (I) 2,2,4,4-tetramethyl-cyclobutane-1,3-diol diacetate, and as only further compound(s) (II) acetic acid, optionally acetic anhydride and optionally zinc chloride;
where the composition is not a mixture comprising as compound of formula (I) 2,2,4,4-tetramethyl-cyclobutane-1,3-diol diformate, and as only further compounds (II) formic acid, benzene, optionally water, optionally sodium bicarbonate and
optionally sodium sulfate;
where the composition is not a mixture comprising as compound of formula (I) 2,2,4,4-tetramethyl-cyclobutane-1,3-diol diisobutyrate, and as only further compounds (II) isobutyric acid, isobutyric anhydride and optionally zinc chloride;
where the composition is not a mixture comprising as compound of formula (I) 2,2,4,4-tetramethyl-cyclobutane-1,3-diol di-n-butyrate, and as only further compound(s) (II) n-butyric acid, optionally n-butyric anhydride and optionally zinc chloride;
where the composition is not a composition consisting of cyclobutane-1,3-diol diacetate and cellulose acetate butyrate;
where the composition is not a composition consisting of cyclobutane-1,3-diol diisobutyrate and cellulose acetate butyrate; and
where the composition is not a composition consisting of cyclobutane-1,3-diol di-n-butyrate and cellulose acetate butyrate.

It was further found that the compounds of the general formula (I) generally exhibit a pleasant and characteristic odor and can be used to produce fragranced ready-to-use compositions. In addition, they can advantageously be combined with other aroma chemicals different from compounds (I) to create new scent profiles.

Therefore, the present invention further relates to a method of preparing a fragranced ready-to-use composition, comprising incorporating at least one compound of formula (I), a stereoisomer thereof or a mixture of stereoisomers thereof, into a ready-to-use composition,
where the ready-to-use composition is not a composition consisting of cyclobutane-1,3-diol diacetate and cellulose acetate butyrate;
where the ready-to-use composition is not a composition consisting of cyclobutane-1,3-diol diisobutyrate and cellulose acetate butyrate; and
where the ready-to-use composition is not a composition consisting of cyclobutane-1,3-diol di-n-butyrate and cellulose acetate butyrate.

The present invention further relates to the use of a compound of formula (I), a stereoisomer thereof or a mixture of stereoisomers thereof, as defined above, for modifying the scent character of a fragranced ready-to-use composition.

Amongst the group of compounds of formula (I), the ether compounds of formula (I.a) and the ester compounds of formula (I.b) have not been described in the art.

Therefore, the present invention also relates to novel compounds of the general formula (I.a) 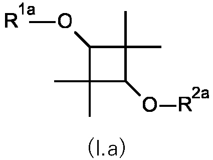 wherein
R^{1a} is C₂-C₄-alkyl and
R^{2a} is hydrogen or C₂-C₄-alkyl,
a stereoisomer thereof or a mixture of stereoisomers thereof and to a method of their preparation.

Furthermore, the present invention relates to novel compounds of the general formula (I.b) 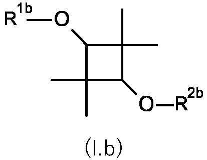 wherein
R^{1b} is -(C=O)-R³,
R^{2b} is hydrogen or -(C=O)-R⁴ and
R³ and R⁴, independently of one another, are selected from the group consisting of hydrogen and C₁-C₄-alkyl, with the provision that R³ and R⁴, if present, are different, a stereoisomer thereof or a mixture of stereoisomers thereof;
except for 2,2,4,4-tetramethylcyclobutanediol monobutyrate;
and to a method of their preparation.

The compounds of formula (I), their stereoisomers or the mixtures of their stereoisomers, possess advantageous organoleptic properties, in particular a pleasant odor. Therefore, they can be favorably used as an aroma chemical for example in perfume composition, cosmetic composition, body care composition, product for oral and dental hygiene, hygiene article, cleaning composition, textile detergent composition, dishwashing compositions, compositions for scent dispensers, food, food supplement, pharmaceutical composition, crop protection composition and other ready-to-use compositions.

By virtue of their physical properties, the compounds of formula (I), their stereoisomers or the mixtures of their stereoisomers, have particularly good, virtually universal solvent properties for other fragrances and other customary ingredients in fragranced ready-to-use compositions such as, in particular, perfume compositions. Therefore, the compounds of formula (I), their stereoisomers or the mixtures of their stereoisomers are favorably combinable with other aroma chemicals, allowing, in particular, the creation of perfume compositions having novel advantageous sensory profiles.

Furthermore, the compounds of formula (I), their stereoisomers or the mixtures of their stereoisomers, can be produced in good yields and purities by a one-step or a two-step synthesis, respectively, starting from readily available and cheap 2,2,4,4-tetramethylcyclobutane-1,3-diol. Thus, the compounds of formula (I), their stereoisomers or the mixtures of their stereoisomers, can be produced in large scales and in a simple and cost-efficient manner.

In addition, the compounds of formula (I), their stereoisomers or the mixtures of their stereoisomers are likely to have low or no toxicity as they are derived from 2,2,4,4-tetramethylcyclobutane-1,3-diol, for which there is no current evidence of carcinogenic or toxic effects.

### DETAILED DESCRIPTION OF THE INVENTION

In the context of the present invention, the expression "C₁-C₄-alkyl" refers to methyl, ethyl, n-propyl, isopropyl, n-butyl, isobutyl and tert.-butyl. Preferably, the expression "C₁-C₄-alkyl" refers to methyl, ethyl, n-propyl and isopropyl, in particular to methyl and ethyl.

The term "aroma chemical" denotes a substance, which is used to obtain a sensory impression, to be more precise an olfactory or flavor impression, in particular a fragrance or flavor impression. Thus, these aroma chemicals are suitable for use in "aroma chemical compositions" and/or "fragranced ready-to-use compositions". Accordingly, the term "aroma chemical composition", as used herein, refers to a composition, which predominately induces an odor impression. Likewise, the term "fragranced ready-to-use composition", as used herein, refers to a ready-to-use composition, which predominately induces an odor impression.

The term "olfactory" denotes an odor impression without any positive or negative judgement, while the term "fragrance" (also termed "perfume" or "scent") is connected to an odor impression which is generally felt as pleasant. A flavor induces a taste impression.

The term "odor-intensive substances" refers to substances or aroma chemicals exhibiting intense odor impressions. Intense odor impressions are to be understood as meaning those properties of aroma chemicals, which permit a striking perception even in very low gas space concentrations. The intensity can be determined via a threshold value determination. A threshold value is the concentration of a substance in the relevant gas space at which an odor impression can just still be perceived by a representative test panel, although it no longer has to be defined. A substance class which probably belongs to the most odor-intensive known substance classes, i.e. has very low odor threshold values, are thiols, whose threshold value is often in the ppb/m³ range.

"Advantageous sensory properties", "advantageous organoleptic properties" or "pleasant odor" are hedonistic expressions, which describe the niceness and conciseness of an odor impression conveyed by an aroma chemical. "Niceness" and "conciseness" are terms which are familiar to the person skilled in the art, a perfumer. Niceness generally refers to a spontaneously brought about, positively perceived, pleasant sensory impression. However, "nice" does not have to be synonymous with "sweet". "Nice" can also be the odor of musk or sandalwood. "Conciseness" generally refers to a spontaneously brought about sensory impression which - for the same test panel - brings about a reproducibly identical reminder of something specific. For example, a substance can have an odor which is spontaneously reminiscent of that of an "apple": the odor would then be concisely of "apples". If this apple odor were very pleasant because the odor is reminiscent, for example, of a sweet, fully ripe apple, the odor would be termed "nice". However, the odor of a typically tart apple can also be concise. If both reactions arise upon smelling the substance, in the example thus a nice and concise apple odor, then this substance has particularly advantageous sensory properties.

Depending on the spatial arrangement of the oxygen atoms relative to the cyclobutane ring, the compounds of the formula (I) can exist as individual cis-isomers (I-cis) or as trans-isomers (I-trans) 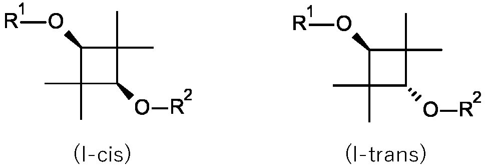 or as cis/trans isomer mixtures.

The present invention thus relates to the use of the cis isomers, i.e. (I-cis), the use of the trans isomers, i.e. (I-trans), and also the use of cis/trans isomer mixtures thereof, i.e. (I-cis/trans). Thus, if not stated otherwise, the expressions "compounds I", "compounds of the general formula I" and the like, as used herein, refers to the pure cis isomers, the pure trans isomers as well as to cis/trans isomer mixtures thereof, in which the isomers are present in equal quantities or contain one of the isomers in excess.

The pure cis isomers and the pure trans isomers as well as the cis/trans isomer mixtures of the compounds (I) have all advantageous organoleptic properties. Thus, the pure cis isomers and the pure trans isomers as well as the cis/trans isomer mixtures of the compounds (I) are equally suitable for use as aroma chemicals. Furthermore, at least for some of the compounds (I), e.g. for the ester compound (3-acetoxy-2,2,4,4-tetramethyl-cyclobutyl) acetate, the organoleptic properties of the cis isomer does not differ significantly from the organoleptic properties of the trans isomer.

On the other hand, the basic scent profile of the ether compounds of the general formula (I) differs from the scent profile of the ester compounds of the general formula (I).

Therefore a first embodiment of the present invention relates to the use of an ether compound of the general formula (I), where in formula (I)
R¹ is C₁-C₄-alkyl and
R² is hydrogen or C₁-C₄-alkyl.

The compounds (I) of this first embodiment, encompass the mono-ethers as well as the di-ethers of 2,2,4,4-tetramethylcyclobutane-1,3-diol. Typically, the mono-ethers of 2,2,4,4-tetramethylcyclobutane-1,3-diol exhibit a less intense odor than the corresponding di-ethers of 2,2,4,4-tetramethylcyclobutane-1,3-diol.

Therefore, the di-ether compounds, i.e. the compounds of the general formula (I), where in formula (I) R¹ and R², independently of one another, are selected from C₁-C₄-alkyl, are preferred for use.

Furthermore, the synthesis of the ether compounds of the general formula (I), where the substituents attached to the oxygen atoms are identical, is generally simpler than the synthesis of the ether compounds of the general formula (I) having different substituents on the oxygen atoms.

Accordingly, further preferred for use are ether compounds of the general formula (I), wherein the radicals R¹ and R² are identical.

Examples of preferred ether compounds of the general formula (I) are
2,4-dimethoxy-1,1,3,3-tetramethyl-cyclobutane,
2,4-dimethoxy-1,1,3,3-tetramethyl-cyclobutane, which is essentially present in the form of its cis isomer,
2,4-dimethoxy-1,1,3,3-tetramethyl-cyclobutane, which is essentially present in the form of its trans isomer,
2,4-diethoxy-1,1,3,3-tetramethyl-cyclobutane,
2,4-diethoxy-1,1,3,3-tetramethyl-cyclobutane, which is essentially present in the form of its cis isomer,
2,4-diethoxy-1,1,3,3-tetramethyl-cyclobutane, which is essentially present in the form of its trans isomer,
2,4-di-n-propoxy-1,1,3,3-tetramethyl-cyclobutane,
2,4-di-n-propoxy-1,1,3,3-tetramethyl-cyclobutane, which is essentially present in the form of its cis isomer,
2,4-di-n-propoxy-1,1,3,3-tetramethyl-cyclobutane, which is essentially present in the form of its trans isomer,
2,4-diisopropoxy-1,1,3,3-tetramethyl-cyclobutane,
2,4-diisopropoxy-1,1,3,3-tetramethyl-cyclobutane, which is essentially present in the form of its cis isomer,
2,4-diisopropoxy-1,1,3,3-tetramethyl-cyclobutane, which is essentially present in the form of its trans isomer,
2,4-di-n-butoxy-1,1,3,3-tetramethyl-cyclobutane,
2,4-di-n-butoxy-1,1,3,3-tetramethyl-cyclobutane, which is essentially present in the form of its cis isomer,
2,4-di-n-butoxy-1,1,3,3-tetramethyl-cyclobutane, which is essentially present in the form of its trans isomer,
2,4-diisobutoxy-1,1,3,3-tetramethyl-cyclobutane,
2,4-diisobutoxy-1,1,3,3-tetramethyl-cyclobutane, which is essentially present in the form of its cis isomer, and
2,4-diisobutoxy-1,1,3,3-tetramethyl-cyclobutane, which is essentially present in the form of its trans isomer,
2,4-di-tert.-butoxy-1,1,3,3-tetramethyl-cyclobutane,
2,4-di-tert.-butoxy-1,1,3,3-tetramethyl-cyclobutane, which is essentially present in the form of its cis isomer, and
2,4-di-tert.-butoxy-1,1,3,3-tetramethyl-cyclobutane, which is essentially present in the form of its trans isomer.

Despite their generally somewhat less intense odor of the mono-ethers of 2,2,4,4-tetramethylcyclobutane-1,3-diol when compared to the corresponding di-ethers of 2,2,4,4-tetramethylcyclobutane-1,3-diol, these mono-ethers have nevertheless characteristic scent profiles. Thus, the mono-ether derivatives of 2,2,4,4-tetramethylcyclobutane-1,3-diol according to the present invention are valuable compounds for use as aroma chemicals.

Accordingly, another preferred embodiment of the present invention relates to the use of a compound of the general formula (I) 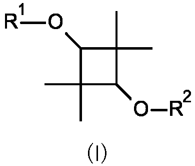 wherein
R¹ is C₁-C₄-alkyl and
R² is hydrogen,
a stereoisomer thereof or a mixture of stereoisomers thereof,
as an aroma chemical.

Examples of preferred mono-ether compounds (I) of this embodiment are
3-methoxy-2,2,4,4-tetramethyl-cyclobutanol,
3-methoxy-2,2,4,4-tetramethyl-cyclobutanol, which is essentially present in the form of its cis isomer,
3-methoxy-2,2,4,4-tetramethyl-cyclobutanol, which is essentially present in the form of its trans isomer,
3-ethoxy-2,2,4,4-tetramethyl-cyclobutanol,
3-ethoxy-2,2,4,4-tetramethyl-cyclobutanol, which is essentially present in the form of its cis isomer,
3-ethoxy-2,2,4,4-tetramethyl-cyclobutanol, which is essentially present in the form of its trans isomer,
3-n-propoxy-2,2,4,4-tetramethyl-cyclobutanol,
3-n-propoxy-2,2,4,4-tetramethyl-cyclobutanol, which is essentially present in the form of its cis isomer,
3-n-propoxy-2,2,4,4-tetramethyl-cyclobutanol, which is essentially present in the form of its trans isomer,
3-n-butoxy-2,2,4,4-tetramethyl-cyclobutanol,
3-n-butoxy-2,2,4,4-tetramethyl-cyclobutanol, which is essentially present in the form of its cis isomer,
3-n-butoxy-2,2,4,4-tetramethyl-cyclobutanol, which is essentially present in the form of its trans isomer,
3-tert.-butoxy-2,2,4,4-tetramethyl-cyclobutanol,
3-tert.-butoxy-2,2,4,4-tetramethyl-cyclobutanol, which is essentially present in the form of its cis isomer, and
3-tert.-butoxy-2,2,4,4-tetramethyl-cyclobutanol, which is essentially present in the form of its trans isomer.

More preferred for use are ether compounds of the general formula (I), wherein R¹ and R² are C₁-C₃-alkyl.

Even more preferred for use are ether compounds of the general formula (I), wherein R¹ and R² are identical and selected from the group consisting of methyl, ethyl, n-propyl and isopropyl.

Particularly preferred for use are ether compounds of the general formula (I), wherein R¹ and R² are methyl or ethyl.

Especially preferred for use are
2,4-dimethoxy-1,1,3,3-tetramethyl-cyclobutane,
2,4-dimethoxy-1,1,3,3-tetramethyl-cyclobutane, which is essentially present in the form of its cis isomer,
2,4-dimethoxy-1,1,3,3-tetramethyl-cyclobutane, which is essentially present in the form of its trans isomer,
2,4-diethoxy-1,1,3,3-tetramethyl-cyclobutane,
2,4-diethoxy-1,1,3,3-tetramethyl-cyclobutane, which is essentially present in the form of its cis isomer, and
2,4-diethoxy-1,1,3,3-tetramethyl-cyclobutane, which is essentially present in the form of its trans isomer.

A second embodiment of the present invention relates to the use of an ester compound of the general formula (I), where in formula (I)
R¹ is -(C=O)-R³,
R² is hydrogen or -(C=O)-R⁴ and
R³ and R⁴ are as defined above.

The compounds (I) of this second embodiment, encompass the mono-esters as well as the di-esters of 2,2,4,4-tetramethylcyclobutane-1,3-diol. Typically, the mono-esters of 2,2,4,4-tetramethylcyclobutane-1,3-diol exhibit a less intense odor than the corresponding di-esters of 2,2,4,4-tetramethylcyclobutane-1,3-diol.

Therefore, the di-ester compounds, i.e. the compounds of the general formula (I), where in formula (I)
R¹ is -(C=O)-R³,
R² is -(C=O)-R⁴ and
R³ and R⁴ are as defined above,
are preferred for use.

Also here, the synthesis of the esters compounds of the general formula (I), where the substituents attached to the oxygen atoms are identical, is generally simpler than the synthesis of the esters compounds of the general formula (I) having different substituents on the oxygen atoms.

Accordingly, further preferred for use are ester compounds of the general formula (I), wherein
R¹ is -(C=O)-R³,
R² is -(C=O)-R⁴, and
R³ and R⁴ are as defined above,
and where the radicals R³ and R⁴ are identical.

Further preferred for use are ester compounds of the general formula (I), wherein R³ and R⁴ are selected from the group consisting of hydrogen and C₁-C₃-alkyl.

Examples of preferred ester compounds of the general formula (I) are
(3-formyloxy-2,2,4,4-tetramethyl-cyclobutyl) formate,
(3-formyloxy-2,2,4,4-tetramethyl-cyclobutyl) formate, which is essentially present **in** the form of its cis isomer,
(3-formyloxy-2,2,4,4-tetramethyl-cyclobutyl) formate, which is essentially present **in** the form of its trans isomer,
(3-acetoxy-2,2,4,4-tetramethyl-cyclobutyl) acetate,
(3-acetoxy-2,2,4,4-tetramethyl-cyclobutyl) acetate, which is essentially present in the form of its cis isomer,
(3-acetoxy-2,2,4,4-tetramethyl-cyclobutyl) acetate, which is essentially present in the form of its trans isomer,
(3-propanoyloxy-2,2,4,4-tetramethyl-cyclobutyl) propanoate,
(3-propanoyloxy-2,2,4,4-tetramethyl-cyclobutyl) propanoate, which is essentially present in the form of its cis isomer,
(3-propanoyloxy-2,2,4,4-tetramethyl-cyclobutyl) propanoate, which is essentially present in the form of its trans isomer,
(3-isobutanoyloxy-2,2,4,4-tetramethyl-cyclobutyl) isobutanoate,
(3-isobutanoyloxy-2,2,4,4-tetramethyl-cyclobutyl) isobutanoate, which is essentially present in the form of its cis isomer, and
(3-isobutanoyloxy-2,2,4,4-tetramethyl-cyclobutyl) isobutanoate, which is essentially present in the form of its trans isomer.

Even more preferred for use are ester compounds of the general formula (I), wherein R³ and R⁴ are identical and selected from the group consisting of hydrogen and C₁-C₃-alkyl.

Particularly preferred for use are ester compounds of the general formula (I), wherein R³ and R⁴ are selected from the group consisting of hydrogen, methyl or ethyl.

Especially preferred for use are
(3-acetoxy-2,2,4,4-tetramethyl-cyclobutyl) acetate,
(3-acetoxy-2,2,4,4-tetramethyl-cyclobutyl) acetate, which is essentially present in the form of its cis isomer, and
(3-acetoxy-2,2,4,4-tetramethyl-cyclobutyl) acetate, which is essentially present in the form of its trans isomer.

In a particular embodiment of the present invention, the compound of the general formula (I) is essentially present in the form of its cis isomer.

In another particular embodiment of the present invention, the compound of the general formula (I) is essentially present in the form of its trans isomers.

Another preferred embodiment of the present invention relates to the use of a compound of the general formula (I) 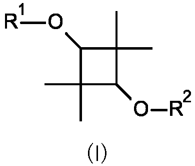 wherein
R¹ is C₁-C₄-alkyl and
R² is -(C=O)-R³,
a stereoisomer thereof or a mixture of stereoisomers thereof,
as an aroma chemical.

Regarding the preferred and very preferred meanings of the radicals C₁-C₄-alkyl and -(C=O)-R³, reference is made to the statements given above.

Particularly preferred compounds of this embodiment are
(3-methoxy-2,2,4,4-tetramethyl-cyclobutyl) acetate,
(3-methoxy-2,2,4,4-tetramethyl-cyclobutyl) acetate, which is essentially present in the form of its cis isomer, and
(3-methoxy-2,2,4,4-tetramethyl-cyclobutyl) acetate, which is essentially present in the form of its trans isomer.

In this connection, the term "essentially" means that the cis or the trans isomer of the particular compound is present in an amount of at least 90% by weight, preferably in an amount of at least 95% by weight, in particular in an amount of at least 98% by weight, based on the total amount of the cis and the trans isomers.

Typically, the compounds (I) are produced as cis/trans isomer mixtures. The separation of the cis and the trans isomers can be difficult and laborious or cannot be achieved completely with reasonable effort.

Thus, a preferred embodiment of the present invention relates to the use of a compound of the general formula (I), as defined above and below, where the compound of the general formula (I) is present in the form of a cis/trans isomer mixture. In these mixtures, the two isomers can be present in equal amounts or almost equal amounts, e.g. in a cis/trans or trans/cis isomer ratio of 50:50 or 55:45, or one of the isomers, i.e. either the cis or the trans isomer, can be present in excess, e.g. one of the isomers can be present in a cis/trans or trans/cis isomer ratio of 60:40, 65:35, 70:30 or 75:25.

Furthermore, the compounds (I) can be used as aroma chemicals over a wide range of purity, as long as the impurities do not have a significant detrimental effect on the scent of the compounds (I). For the use as aroma chemicals, according to the invention, the purity of the compounds (I) is therefore not specifically limited. Preferably, the compounds (I) have a purity of at least 70%, in particular of at least 90% and especially of at least 95%.

Due to the method of their preparation starting from 2,2,4,4-tetramethylcyclobutane-1,3-diol, the di-ether compounds of the general formula (I) may comprise minor amounts of mono-ether compounds (I-OH) and the di-ester compounds of the general formula (I) may comprise minor amount of mono-ester compounds (II-OH) 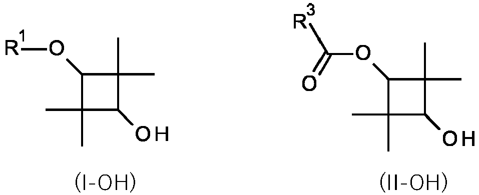 wherein R¹ and R³ have one of the meaning given above.

Preferably, the amounts of mono-substituted compounds (I-OH) or (II-OH), which can be comprised in the di-ether and the di-ester compounds of the general formula (I) are less than 25% by weight, more preferably less than 10% by weight, even more preferably less than 5% by weight, in particular less than 1% by weight, based on the total amount of the compound (I).

In a particularly preferred embodiment of the present invention the di-ether and diester compounds of the general formula (I) do not comprise mono-substituted compounds (I-OH) or (II-OH), respectively.

Likewise, the mono-ether and the mono-ester compounds of the general formula (I) may comprise minor amounts of the corresponding di-substituted compounds. Preferably, the amounts of di-substituted compounds of the general formula (I), which can be comprised in the mono-ether and the mono-ester compounds of the general formula (I) are less than 25% by weight, more preferably less than 10% by weight, even more preferably less than 5% by weight, in particular less than 1% by weight, based on the total amount of the compound (I).

Furthermore, the di-ether compounds of the general formula (I), wherein the radicals R¹ and R² are different, as well as the di-ester compounds of the general formula (I), wherein the radicals R³ and R⁴ are different, hereinafter referred to as unsymmetrically substituted compounds, may comprise minor amounts of the corresponding symmetrically substituted compounds, i.e. di-ether compounds of the general formula (I), wherein the radicals R¹ and R² are identical, and di-esters compounds of the general formula (I), wherein the radicals R³ and R⁴ are identical. Typically, the amounts of symmetrically substituted compounds, which can be comprised in the unsymmetrically substituted compounds of the general formula (I) are less than 50% by weight, preferably less than 25% by weight, more preferably less than 10% by weight, even more preferably less than 5% by weight, in particular less than 1% by weight, based on the total amount of the compound (I).

Furthermore, the ether-ester-compounds of the general formula (I), wherein the radical R¹ is C₁-C₄-alykl and R² is -(C=O)-R³, hereinafter also referred to as mixed substituted compounds (I), may comprise the corresponding di-ether or di-ester compounds. Furthermore, depending on whether the mono-ether or the mono-ester compound is used as the starting material, these mixed substituted compounds (I) can also comprise minor amounts of the corresponding mono-ether or mono-ester compound. Preferably, the amounts of these by-products, which can be comprised in the mixed substituted compounds of the general formula (I) are less than 50% by weight, more preferably less than 40% by weight, in particular less than 30% by weight, based on the total amount of the compound (I).

The aforesaid preferred embodiments can be combined with one another as desired.

Accordingly, in a particular preferred embodiment of the present invention relates to the use of a compound of the general formula (I), which has a purity of at least 95% and is present in the form of a cis/trans isomer mixture.

The mono-ether compounds of the general formula (I) can efficiently be prepared by alkylating 2,2,4,4-tetramethylcyclobutane-1,3-diol using the alkylation reagent R¹-X, wherein R¹ has one of the meanings given above and X represents a leaving group, selected from halogen, such as Cl, Br, I, and sulfonates, such as tosylate, mesylate, triflate or nonaflate, typically in the presence of a base.

Suitable bases are typically selected from inorganic bases and organic bases.

Suitable inorganic bases that can be used in this alkylation reaction are for example alkali metal carbonates, e.g. Li₂CO₃, Na₂CO₃, K₂CO₃ or Cs₂CO₃, alkali metal hydroxides, e.g. LiOH, NaOH or KOH, and hydride donors, e.g. NaH, LiAlH₄ or NaBH₄.

Suitable organic bases that can be used in this alkylation reaction are for example tertiary amines, e.g. trimethylamine, triethylamine, tripropylamine, ethyldiisopropylamine and the like, or basic N-heterocycles, such as morpholine, pyridine, lutidine, DMAP, DABCO, DBU or DBN.

The alkylation reaction is performed under conventional alkylation reaction conditions that are well known to the skilled person.

Typically, 2,2,4,4-tetramethylcyclobutane-1,3-diol is reacted with sub-equimolar or almost equimolar amounts, e.g. 0.7, 0.8, 0.9, or 0.95 equivalents, of the alkylation reagent R¹-X to give the mono-substituted compound (I-OH), which is further subjected to a purification step in order to remove unwanted by-products or impurities, such as residual starting material or the corresponding di-substituted compounds.

Generally, the purification step is performed by using common purification methods, such as crystallization, distillation or chromatographic methods, e.g. column chromatography or high performance liquid chromatography.

The preparation of di-ether compounds of the general formula (I), where the radicals R¹ and R² are identical, is typically performed by reacting 2,2,4,4-tetramethylcyclobutane-1,3-diol with at least two equivalents, e.g. 2.0, 2.1, 2.5 or 3.0 equivalents, of the alkylation reagent R¹-X or R²-X, respectively, wherein the radicals R¹, R² and X have one of the meanings given above, and the obtained raw product is subsequently subjected to a purification step, as defined above. In case where only minor amounts of impurities and by-products are present in the raw-product mixture the purification step can also be performed via a simple extractive workup.

The preparation of di-ether compounds of the general formula (I), where the radicals R¹ and R² are different, is generally performed by first reacting 2,2,4,4-tetramethylcyclobutane-1,3-diol with sub-equimolar or almost equimolar amounts, e.g. 0.7, 0.8, 0.9, or 0.95 equivalents, of the alkylation reagent R¹-X to give the mono-substituted compound (I-OH), which after a purification step is further reacted with the second alkylation reagent R²-X.

Generally, the ester compounds of the general formula (I) can efficiently be prepared by reacting 2,2,4,4-tetramethylcyclobutane-1,3-diol with the carboxylic acid R³-COOH and optionally with the carboxylic acid R⁴-COOH, wherein R³ and R⁴ have one of the meanings given above, or an acid anhydride thereof, or a mixture of the carboxylic acid R³-COOH and/or R⁴-COOH with an acid anhydride thereof. The reaction is typically performed in the presence of an esterification catalyst or a base.

Suitable esterification catalysts that can be applied in this reaction are well known to the skilled person. Suitable esterification catalysts are for example metal based catalysts, e.g. iron, cadmium, cobalt, lead, zinc, antimony, magnesium, titanium and tin catalysts in the form of metals, metal oxides or metal salts, such as metal alcoxylates; mineral acids, such as sulfuric acid, hydrochloric acid or phosphoric acid; or organic sulfonic acids, such as methane sulfonic acid or para-toluene sulfonic acid.

Suitable bases are for example organic bases, as defined above.

Alternatively, the ester compounds of the general formula (I) can be prepared by reacting 2,2,4,4-tetramethylcyclobutane-1,3-diol with an acid halogenide of the formulae R³-(C=O)X' or R⁴-(C=O)X', wherein R³ and R⁴ have one of the meanings given above and X' is halogen, such as Cl, Br or I, in the presence of an organic base.

Suitable organic bases are as defined above.

The individual reaction conditions for the preparation of the ester compounds of the general formula (I) are well known to the skilled person.

For the preparation of the mono-ester compounds of the general formula (I), 2,2,4,4-tetramethylcyclobutane-1,3-diol is typically reacted with sub-equimolar or almost equimolar amounts, e.g. 0.7, 0.8, 0.9, or 0.95 equivalents, of the carboxylic acid R³-COOH or an anhydride thereof or a mixture of said carboxylic acid with an anhydride thereof. Alternatively, 2,2,4,4-tetramethylcyclobutane-1,3-diol is typically reacted with the same amounts of the acid halogenide R³-(C=O)X' in the presence of an organic base. The obtained raw product is subsequently subjected to a purification step, as defined above.

For the preparation of di-ester compounds of the general formula (I), where the radicals R³ and R⁴ are identical, 2,2,4,4-tetramethylcyclobutane-1,3-diol is typically reacted with at least two equivalents, e.g. 2.0, 2.1, 2.5 or 3.0 equivalents, of the carboxylic acid R³-COOH or R⁴-COOH, respectively, an anhydride thereof or a mixture of said carboxylic acid with an anhydride thereof. Alternatively, 2,2,4,4-tetramethylcyclobutane-1,3-diol is typically reacted with at least two equivalents of the acid halogenide R³-(C=O)X' or R⁴-(C=O)X', respectively, in the presence of an organic base. The obtained raw product is subsequently subjected to a purification step, as defined above. In case where only minor amounts of impurities and by-products are present in the raw-product mixture the purification step can also be performed via a simple extractive workup.

The preparation of di-ester compounds of the general formula (I), where the radicals R³ and R⁴ are different, is generally performed by first reacting 2,2,4,4-tetramethylcyclobutane-1,3-diol with sub-equimolar or almost equimolar amounts, e.g. 0.7, 0.8, 0.9, or 0.95 equivalents, of the carboxylic acid R³-COOH, or an anhydride thereof, to give the mono-substituted compound (II-OH), which after a purification step is further reacted with a second carboxylic acid R⁴-COOH, or an anhydride thereof.

Alternatively, the preparation of compounds (I), where the radicals R³ and R⁴ are different, can also be achieved by first reacting 2,2,4,4-tetramethylcyclobutane-1,3-diol with sub-equimolar or almost equimolar amounts, e.g. 0.7, 0.8, 0.9, or 0.95 equivalents, of the acid halogenide R³-(C=O)X' in the presence of an organic base to give the mono-substituted compound (II-OH), which after a purification step is further reacted with a second acid halogenide R⁴-(C=O)X'.

The starting material 2,2,4,4-tetramethylcyclobutane-1,3-diol is a diol building block that is frequently used as a monomer for the synthesis of polymeric materials. Thus, 2,2,4,4-tetramethylcyclobutane-1,3-diol is readily available from commercial sources. Alternatively, 2,2,4,4-tetramethylcyclobutane-1,3-diol can also by synthesized in large quantities using processes that are well described in the art, e.g. via the dimerization of dimethylketene.

### Compositions:

The compounds of formula (I), the stereoisomers thereof or a mixture of stereoisomers thereof can be used in a wide range of compositions, such as ready-to-use compositions. The olfactory properties, the substance properties (such as solubility in customary solvents and compatibility with further customary constituents of such compositions), as well as the toxicological acceptability of the compounds of formula (I), underline their particular suitability for the stated use purposes and compositions.

Furthermore, the compounds of the general formula (I) exhibit advantageous secondary properties.

For example, the compounds of the general formula (I) can provide better sensory profiles as a result of synergistic effects with other fragrances, which means that they can provide a booster effect for other fragrances. They are therefore suitable as boosters for other fragrances.

Booster effect means that the substances enhance and intensify, in perfumery formulations, the overall impression of the mixture. In the mint range, for example, it is known that menthyl methyl ether intensifies the perfumery or taste mixtures of peppermint oils and particularly in top notes brings about a considerably more intensive and more complex perception although the ether itself, being a pure substance, develops no particular intensive odor at all. In fragrance applications, Hedione^{®} (methyl dihydrojasmonate), which as a pure substance only exhibits a light floral jasmin-note, reinforces diffusion, freshness and volume of a perfume composition as an odor booster. Booster effects are particularly desired when top-note-characterized applications are required, in which the odor impression is to be conveyed particularly quickly and intensively, for example in deodorants, air fresheners or in the taste sector in chewing gums.

To achieve such a booster effect, the compounds of the general formula (I), the stereoisomers thereof or a mixture of stereoisomers thereof are generally used in an amount of 0.1 - 20% by weight, preferably in an amount of 0.5 to 5% by weight, in particular in an amount of from 0.6 to 3% by weight, based on the total weight of the fragrance mixture.

Furthermore, the compounds of the general formula (I), the stereoisomers thereof or a mixture of stereoisomers thereof can have further positive effects on the composition itself, where they are applied in. For example, the compounds of the general formula (I), the stereoisomers thereof or a mixture of stereoisomers thereof can enhance the overall performance of the composition, such as the stability, e.g. the formulation stability, the extendability or the staying power of the composition.

Accordingly, a further embodiment of the invention therefore relates to the use of a compound of the general formula (I), or a mixture of two or more different compounds of formula (I) or a stereoisomer thereof or a mixture of stereoisomers thereof, as an aroma chemical in a ready-to-use composition.

The term "ready-to-use composition", as used herein, refers to composition, which is intended to be applied or used on its own by the final user.

Generally, the ready-to-use composition, in which the aroma chemicals of the present invention, i.e. the compounds of the general formula (I), as defined above, can be applied, are fragranced ready-to-use compositions.

Fragranced ready-to-use composition, in which the aroma chemicals of the present invention, i.e. the compounds of the general formula (I), as defined above, can be applied, are for example compositions used in personal care, compositions used in home care, compositions used in industrial applications as well as compositions used in other applications, such as pharmaceutical compositions or crop protection compositions.

Preferably, the ready-to-use compositions are selected from perfume composition, cosmetic composition, body care composition, product for oral and dental hygiene, hygiene article, cleaning composition, textile detergent composition, dishwashing compositions, compositions for scent dispensers, food, food supplement, pharmaceutical composition and crop protection composition.

Perfume compositions can be selected from fine fragrances, air fresheners in liquid form, gel-like form or a form applied to a solid carrier, aerosol sprays, scented cleaners, perfumed candles and oils, such as lamp oils or oils for massage.

Exemples for fine fragrances are perfume extracts, Eau de Parfums, Eau de Toilettes, Eau de Colognes, Eau de Solide and Extrait Parfum and the like.

Body care compositions can be selected from aftershaves, pre-shave products, splash colognes, solid and liquid soaps, shower gels, shampoos, shaving soaps, shaving foams, bath oils, cosmetic emulsions of the oil-in-water type, of the water-in-oil type and of the water-in-oil-in-water type, such as e.g. skin creams and lotions, face creams and lotions, sunscreen creams and lotions, after-sun creams and lotions, hand creams and lotions, foot creams and lotions, hair removal creams and lotions, aftershave creams and lotions and tanning creams and lotions, powders, hydrogels, hair care products, such as e.g. hairsprays, hair gels, setting hair lotions, hair conditioners, hair shampoo and permanent and semi-permanent hair colorants, hair shaping compositions, such as cold waves and hair smoothing compositions, hair tonics, hair creams and hair lotions, deodorants and antiperspirants, such as e.g. underarm sprays, roll-ons, deodorant sticks and deodorant creams, products of decorative cosmetics, such as e.g. eye-liners eye-shadows, nail varnishes, make-ups, lipsticks and mascara.

Products for oral and dental hygiene can be selected from toothpaste, dental floss, mouth wash, breath fresheners, dental foam, dental gels and dental strips.

Hygiene articles can be selected from joss sticks, insecticides, repellents, propellants, rust removers, perfumed freshening wipes, armpit pads, baby diapers, sanitary towels, toilet paper, cosmetic wipes, pocket tissues, dishwasher and deodorizer.

Cleaning compositions, such as e.g. cleaners for solid surfaces can be selected from perfumed acidic, alkaline and neutral cleaners, such as e.g. floor cleaners, window cleaners, bath and sanitary cleaners, scouring milk, solid and liquid toilet cleaners, powder and foam carpet cleaners, dishwashing detergents for hand and machine dishwashing, waxes and polishes such as furniture polishes, floor waxes, shoe creams, disinfectants, surface disinfectants and sanitary cleaners, brake cleaners, pipe cleaners, limescale removers, grill and oven cleaners, algae and moss removers, mold removers and facade cleaners.

Textile detergent compositions can be selected from liquid detergents, powder detergents, laundry pretreatments such as bleaches, soaking agents and stain removers, fabric softeners, washing soaps, washing tablets.

Food means a raw, cooked, or processed edible substance, ice, beverage or ingredient used or intended for use in whole or in part for human consumption, or chewing gum, gummies, jellies, and confectionaries.

A food supplement is a product intended for ingestion that contains a dietary ingredient intended to add further nutritional value to the diet. A dietary ingredient may be one, or any combination, of the following substances: a vitamin, a mineral, an herb or other botanical, an amino acid, a dietary substance for use by people to supplement the diet by increasing the total dietary intake, a concentrate, metabolite, constituent, or extract. Food supplements may be found in many forms such as tablets, capsules, softgels, gelcaps, liquids, or powders.

Pharmaceutical compositions comprise compositions, which are intended for use in the diagnosis, cure, mitigation, treatment, or prevention of disease as well as articles (other than food) intended to affect the structure or any function of the body of man or other animals.

Crop protection compositions comprise compositions, which are intended for the managing of plant diseases, weeds and other pests (both vertebrate and invertebrate) that damage agricultural crops and forestry.

The compositions according to the invention can further comprise one or more substances, such as, for example: preservatives, abrasives, anti-acne agents, agents to combat skin aging, antibacterial agents, anti-cellulite agents, antidandruff agents, anti-inflammatory agents, irritation-preventing agents, irritation-alleviating agents, antimicrobial agents, antioxidants, astringents, sweat-inhibiting agents, antiseptics, antistatics, binders, buffers, carrier materials, chelating agents, cell stimulants, cleaning agents, care agents, hair removal agents, surface-active substances, deodorizing agents, antiperspirants, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant sub-stances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, oils, waxes, fats, phospholipids, saturated fatty acids, mono or polyunsaturated fatty acids, ***α*** -hydroxy acids, polyhydroxy fatty acids, liquefiers, dyes, color-protection agents, pigments, anticorrosives, polyols, surfactants, electrolytes, organic solvents or silicone derivatives.

As mentioned above, it was further found that the compounds of the general formula (I) generally exhibit a pleasant and characteristic odor and can be used to produce fragranced ready-to-use compositions and/or they can advantageously be combined with other aroma chemicals different from compounds (I) to create new scent profiles.

Accordingly, a specific embodiment of the present invention relates to the use of a compound of formula (I), or a mixture of two or more different compounds of formula (I) or a stereoisomer thereof or a mixture of stereoisomers thereof for modifying the scent character of a fragranced ready-to-use composition.

The present invention further relates to aroma chemical compositions comprising at least one compound of formula (I), a stereoisomer thereof or a mixture of stereoisomers thereof, as defined above, and at least one further compound selected from the group consisting of further aroma chemicals different from compounds (I) and non-aroma chemical carriers.

This includes aroma chemical compositions comprising at least one compound of formula (I), a stereoisomer thereof or a mixture of stereoisomers thereof, as defined above, and at least one further aroma chemical different from compounds (I).

Further included are aroma chemical compositions comprising at least one compound of formula (I), a stereoisomer thereof or a mixture of stereoisomers thereof, as defined above, and at least one non-aroma chemical carrier.

Also included are aroma chemical compositions comprising at least one compound of formulae (I), a stereoisomer thereof or a mixture of stereoisomers thereof, as defined above, at least one further aroma chemical different from compounds (I) and at least one non-aroma chemical carrier.

Preferably, the aroma chemical compositions comprise at least one compound of formulae (I), a stereoisomer thereof or a mixture of stereoisomers thereof, as defined above, at least one further aroma chemical different from compounds (I) and at least one non-aroma chemical carrier.

The further aroma chemical different from compounds (I) can for example be one, preferably 2, 3, 4, 5, 6, 7, 8 or further aroma chemicals, selected from the group consisting of:
Geranyl acetate (3,7-Dimethyl-2,6 octadien-1yl acetate), alpha-hexylcinnamaldehyde, 2-phenoxyethyl isobutyrate (Phenirat¹), dihydromyrcenol (2,6-dimethyl-7-octen-2-ol), methyl dihydrojasmonate (preferably with a content of cis isomer of more than 60% by weight) (Hedione⁹, Hedione HC⁹), 4,6,6,7,8,8-hexamethyl-1,3,4,6,7,8-hexahydro-cyclopenta[g]benzopyran (Galaxolid³), tetrahydrolinalool (3,7-dimethyloctan-3-ol), ethyllinalool, benzyl salicylate, 2-methyl-3-(4-tert-butylphenyl)propanal (Lilial²), cinnamyl alcohol, 4,7-methano-3a,4,5,6,7,7a-hexahydro-5-indenyl acetate and/or 4,7-methano-3a,4,5,6,7,7a-hexahydro-6-indenyl acetate (Herbaflorat¹), citronellol, citronellyl acetate, tetrahydrogeraniol, vanillin, linalyl acetate, styrolyl acetate (1-phenylethyl acetate), octahydro-2,3,8,8-tetramethyl-2-acetonaphthone and/or 2-acetyl-1,2,3,4,6,7,8-octahydro-2,3,8,8-tetramethylnaphthalene (Iso E Super³), hexyl salicylate, 4-tert-butylcyclohexyl acetate (Oryclone¹), 2-tert-butylcyclohexyl acetate (Agrumex HC¹), alpha-ionone (4-(2,2,6-trimethyl-2-cyclohexen-1-yl)-3-buten-2-one), n-alpha-methylionone, alpha-isomethylionone, coumarin, terpinyl acetate, 2-phenylethyl alcohol, 4-(4-hydroxy-4-methylpentyl)-3-cyclohexenecarboxaldehyde (Lyral³), alpha-amylcinnamaldehyde, ethylene brassylate, (E)- and/or (Z)-3-methylcyclopentadec-5-enone (Muscenon⁹), 15-pentadec-11-enolide and/or 15-pentadec-12-enolide (Globalide¹), 15-cyclopentadecanolide (Macrolide¹), 1-(5,6,7,8-tetrahydro-3,5,5,6,8,8-hexamethyl-2-naphthalenyl)ethanone (Tonalid¹⁰), 2-isobutyl-4-methyltetrahydro-2H-pyran-4-ol (Florol⁹), 2-ethyl-4-(2,2,3-trimethyl-3-cyclopenten-1-yl)-2-buten-1-ol (Sandolen¹), cis-3-hexenyl acetate, trans-3-hexenyl acetate, trans-2/cis-6-nonadienol, 2,4-dimethyl-3-cyclohexenecarboxaldehyde (Vertocitral¹), 2,4,4,7-tetramethyloct-6-en-3-one (Claritone¹), 2,6-dimethyl-5-hepten-1-al (Melonal²), borneol, 3-(3-isopropylphenyl)butanal (Florhydral²), 2-methyl-3-(3,4-methylenedioxyphenyl)propanal (Helional³), 3-(4-ethylphenyl)-2,2-dimethylpropanal (Florazon¹), 7-methyl-2H-1,5-benzodioxepin-3(4H)-one (Calone), 3,3,5-trimethylcyclohexyl acetate (preferably with a content of cis isomers of 70% by weight) or more and 2,5,5-trimethyl-1,2,3,4,4a,5,6,7-octahydronaphthalen-2-ol (Ambrinol S¹).

Where trade names are given above, these refer to the following sources:
¹ trade name of Symrise GmbH, Germany;
² trade name of Givaudan AG, Switzerland;
³ trade name of International Flavors & Fragrances Inc., USA;
⁵ trade name of Danisco Seillans S.A., France;
⁹ trade name of Firmenich S.A., Switzerland;
¹⁰ trade name of PFW Aroma Chemicals B.V., the Netherlands.

Further aroma chemicals with which the compounds of formula (I), the stereoisomers thereof or a mixture of stereoisomers thereof, as defined above, can be combined e.g. to give a composition according to the invention can be found e.g. in S. Arctander, Perfume and Flavor Chemicals, Vol. I and II, Montclair, N. J., 1969, self-published or K. Bauer, D. Garbe and H. Surburg, Common Fragrance and Flavor Materials, 4th Ed., Wiley- VCH, Weinheim 2001. Specifically, mention may be made of:
extracts from natural raw materials such as essential oils, concretes, absolutes, resins, resinoids, balsams, tinctures such as e.g.
ambergris tincture; amyris oil; angelica seed oil; angelica root oil; aniseed oil; valerian oil; basil oil; tree moss absolute; bay oil; mugwort oil; benzoin resin; bergamot oil; beeswax absolute; birch tar oil; bitter almond oil; savory oil; buchu leaf oil; cabreuva oil; cade oil; calmus oil; camphor oil; cananga oil; cardamom oil; cascarilla oil; cassia oil; cassia absolute; castoreum absolute; cedar leaf oil; cedar wood oil; cistus oil; citronella oil; lemon oil; copaiba balsam; copaiba balsam oil; coriander oil; costus root oil; cumin oil; cypress oil; davana oil; dill weed oil; dill seed oil; Eau de brouts absolute; oak moss absolute; elemi oil; tarragon oil; eucalyptus citriodora oil; eucalyptus oil; fennel oil; pine needle oil; galbanum oil; galbanum resin; geranium oil; grapefruit oil; guaiacwood oil; gurjun balsam; gurjun balsam oil; helichrysum absolute; helichrysum oil; ginger oil; iris root absolute; iris root oil; jasmine absolute; calmus oil; camomile oil blue; roman camomile oil; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linalool oil; litsea cubeba oil; laurel leaf oil; mace oil; marjoram oil; mandarin oil; massoia bark oil; mimosa absolute; musk seed oil; musk tincture; clary sage oil; nutmeg oil; myrrh absolute; myrrh oil; myrtle oil; clove leaf oil; clove flower oil; neroli oil; olibanum absolute; olibanum oil; opopanax oil; orange blossom absolute; orange oil; origanum oil; palmarosa oil; patchouli oil; perilla oil; peru balsam oil; parsley leaf oil; parsley seed oil; petitgrain oil; peppermint oil; pepper oil; pimento oil; pine oil; pennyroyal oil; rose absolute; rose wood oil; rose oil; rosemary oil; Dalmatian sage oil; Spanish sage oil; sandalwood oil; celery seed oil; spike-lavender oil; star anise oil; styrax oil; tagetes oil; fir needle oil; tea tree oil; turpentine oil; thyme oil; tolubalsam; tonka absolute; tuberose absolute; vanilla extract; violet leaf absolute; verbena oil; vetiver oil; juniper berry oil; wine lees oil; wormwood oil; winter green oil; hyssop oil; civet absolute; cinnamon leaf oil; cinnamon bark oil, and fractions thereof, or ingredients isolated therefrom;
individual fragrances from the group of hydrocarbons, such as e.g. 3-carene; alpha-pinene; beta-pinene; alpha-terpinene; gamma-terpinene; p-cymene; bisabolene; camphene; caryophyllene; cedrene; farnesene; limonene; longifolene; myrcene; ocimene; valencene; (E,Z)-1,3,5-undecatriene; styrene; diphenylmethane;
aliphatic alcohols such as e.g. hexanol; octanol; 3-octanol; 2,6-dimethylheptanol; 2-methyl-2-heptanol; 2-methyl-2-octanol; (E)-2-hexenol; (E)- and (Z)-3-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
aliphatic aldehydes and acetals thereof such as e.g. hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10-trimethyl-5,9-undecadienal; heptanal diethylacetal; 1,1-dimethoxy-2,2,5-trimethyl-4-hexene; citronellyloxyacetaldehyde; (E/Z)-1-(1-methoxypropoxy)-hex-3-ene; the aliphatic ketones and oximes thereof such as e.g. 2-heptanone; 2-octanone; 3-octanone; 2-nonanone; 5-methyl-3-heptanone; 5-methyl-3-heptanone oxime; 2,4,4,7-tetramethyl-6-octen-3-one; 6-methyl-5-hepten-2-one;
aliphatic sulfur-containing compounds such as e.g. 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
taliphatic nitriles such as e.g. 2-nonenenitrile; 2-undecenenitrile; 2-tridecenenitrile; 3,12-tridecadienenitrile; 3,7-dimethyl-2,6-octadienenitrile; 3,7-dimethyl-6-octenenitrile;
esters of aliphatic carboxylic acids such as e.g. (E)- and (Z)-3-hexenyl formate; ethyl acetoacetate; isoamyl acetate; hexyl acetate; 3,5,5-trimethylhexyl acetate;
3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octinate; methyl 2-noninate; allyl 2-isoamyloxy acetate; methyl-3,7-dimethyl-2,6-octadienoate; 4-methyl-2-pentyl crotonate;
acyclic terpene alcohols such as e.g. geraniol; nerol; linalool; lavandulol; nerolidol; farnesol; tetrahydrolinalool; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and the formates, acetates, propionates, isobutyrates, butyrates, isovalerates, pentanoates, hexanoates, crotonates, tiglinates and 3-methyl-2-butenoates thereof;
acyclic terpene aldehydes and ketones such as e.g. geranial; neral; citronellal; 7-hydroxy-3,7-dimethyloctanal; 7-methoxy-3,7-dimethyloctanal; 2,6,10-trimethyl-9-undecenal; geranyl acetone; as well as the dimethyl- and diethylacetals of geranial, neral, 7-hydroxy-3,7-dimethyloctanal; the cyclic terpene alcohols such as e.g. menthol; isopulegol; alpha-terpineol; terpine-4-ol; menthan-8-ol; menthan-1-ol; menthan-7-ol; borneol; isoborneol; linalool oxide; nopol; cedrol; ambrinol; vetiverol; guajol; and the formates, acetates, propionates, isobutyrates, butyrates, isovalerates, pentanoates, hexanoates, crotonates, tiglinates and 3-methyl-2-butenoates thereof;
cyclic terpene aldehydes and ketones such as e.g. menthone; isomenthone; 8-mercaptomenthan-3-one; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methanonaphthalene-8(5H)-one; 2-methyl-4-(2,6,6-trimethyl-1-cyclohexen-1-yl)-2-butenal; nootkatone; dihydronootkatone; 4,6,8-megastigmatrien-3-one; alpha-sinensal; beta-sinensal; acetylated cedar wood oil (methyl cedryl ketone);
cyclic alcohols such as e.g. 4-tert-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
cycloaliphatic alcohols such as e.g. alpha-3,3-trimethylcyclohexylmethanol; 1-(4-isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
cyclic and cycloaliphatic ethers such as e.g. cineol; cedryl methyl ether; cyclododecyl methyl ether; 1,1-dimethoxycyclododecane; (ethoxymethoxy)cyclododecane; alpha-cedrene epoxide; 3a,6,6,9a-tetramethyldodecahydronaphtho[2,1-b]furan; 3a-ethyl-6,6,9a-trimethyldodecahydronaphtho[2,1-b]furan; 1,5,9-trimethyl-13-oxabicyclo-[10.1.0]trideca-4,8-diene; rose oxide; 2-(2,4-dimethyl-3-cyclohexen-1-yl)-5-methyl-5-(1-methylpropyl)-1,3-dioxane;
cyclic and macrocyclic ketones such as e.g. 4-tert-butylcyclohexanone; 2,2,5-trimethyl-5-pentylcyclopentanone; 2-heptylcyclopentanone; 2-pentylcyclo-pentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclohexadecen-1-one; 6,7-dihydro-1,1,2,3,3-pentamethyl-4(5H)-indanone; 8-cyclohexadecen-1-one; 7-cyclohexadecen-1-one; (7/8)-cyclohexadecen-1-one; 9-cycloheptadecen-1-one; cyclopentadecanone; cyclohexadecanone;
cycloaliphatic aldehydes such as e.g. 2,4-dimethyl-3-cyclohexenecarbaldehyde; 2-methyl-4-(2,2,6-trimethylcyclohexen-1-yl)-2-butenal; 4-(4-hydroxy-4-methylpentyl)-3-cyclohexene carbaldehyde; 4-(4-methyl-3-penten-1-yl)-3-cyclohexenecarbaldehyde;
cycloaliphatic ketones such as e.g. 1-(3,3-dimethylcyclohexyl)-4-penten-1-one; 2,2-dimethyl-1-(2,4-dimethyl-3-cyclohexen-1-yl)-1-propanone; 1-(5,5-dimethyl-1-cyclohexen-1-yl)-4-penten-1-one; 2,3,8,8-tetramethyl-1,2,3,4,5,6,7,8-octahydro-2-naphthalenyl methyl ketone; methyl 2,6,10-trimethyl-2,5,9-cyclododecatrienyl ketone; tert-butyl (2,4-dimethyl-3-cyclohexen-1-yl) ketone;
esters of cyclic alcohols such as e.g. 2-tert-butylcyclohexyl acetate; 4-tert-butylcyclohexyl acetate; 2-tert-pentylcyclohexyl acetate; 4-tert-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate;
decahydro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6-indenyl propionate; 4,7-methano-3a,4,5,6,7,7a-hexahydro-5 or 6-indenyl isobutyrate; 4,7-methanooctahydro-5 or 6-indenyl acetate;
esters of cycloaliphatic alcohols such as e.g. 1-cyclohexylethyl crotonate;
esters of cycloaliphatic carboxylic acids such as e.g. allyl 3-cyclohexylpropionate; allyl cyclohexyloxyacetate; cis- and trans-methyl dihydrojasmonate; cis- and trans-methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl 2-methyl-1,3-dioxolane-2-acetate;
araliphatic alcohols such as e.g. benzyl alcohol; 1-phenylethyl alcohol, 2-phenylethyl alcohol, 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;
esters of araliphatic alcohols and aliphatic carboxylic acids such as e.g. benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;
araliphatic ethers such as e.g. 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m-dioxine;
aromatic and araliphatic aldehydes such as e.g. benzaldehyde; phenylacetaldehyde; 3-phenylpropanal; hydratropaaldehyde; 4-methylbenzaldehyde; 4-methylphenyl-acetaldehyde; 3-(4-ethylphenyl)-2,2-dimethylpropanal; 2-methyl-3-(4-isopropylphenyl)-propanal; 2-methyl-3-(4-tert-butylphenyl)propanal; 2-methyl-3-(4-isobutylphenyl)-propanal; 3-(4-tert-butylphenyl)propanal; cinnamaldehyde; alpha-butylcinnamaldehyde; alpha-amylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
aromatic and araliphatic ketones such as e.g. acetophenone; 4-methylacetophenone; 4-methoxyacetophenone; 4-tert-butyl-2,6-dimethylaceto-phenone; 4-phenyl-2-butanone; 4-(4-hydroxyphenyl)-2-butanone; 1-(2-naphthalenyl)ethanone; 2-benzofuranyl-ethanone; (3-methyl-2-benzofuranyl)ethanone; benzophenone; 1,1,2,3,3,6-hexamethyl-5-indanyl methyl ketone; 6-tert-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;
aromatic and araliphatic carboxylic acids and esters thereof such as e.g. benzoic acid; phenylacetic acid; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;
nitrogen-containing aromatic compounds such as e.g. 2,4,6-trinitro-1,3-dimethyl-5-tert-butylbenzene; 3,5-dinitro-2,6-dimethyl-4-tert-butylacetophenone; cinnamonitrile; 3-methyl-5-phenyl-2-pentenonitrile; 3-methyl-5-phenylpentanonitrile; methyl anthranilate; methyl-N-methylanthranilate; Schiff bases of methyl anthranilate with 7-hydroxy-3,7-dimethyloctanal, 2-methyl-3-(4-tert-butylphenyl)propanal or 2,4-dimethyl-3-cyclohexenecarbaldehyde; 6-isopropylquinoline; 6-isobutylquinoline; 6-sec-butylquinoline; 2-(3-phenylpropyl)pyridine; indole; skatole; 2-methoxy-3-isopropylpyrazine; 2-isobutyl-3-methoxypyrazine;
phenols, phenyl ethers and phenyl esters such as e.g. estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresyl phenylacetate;
heterocyclic compounds such as e.g. 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
lactones such as e.g. 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cis- and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

The at least one non-aroma chemical carrier can be a compound, a mixture of compounds or other additives, which have no or no noteworthy sensory properties. Typically, the at least one non-aroma chemical carrier, if present in the aroma chemical compositions according to the present invention, is a compound, a mixture of compounds or other additives, which have no or no noteworthy sensory properties. The non-aroma chemical carrier serves for the dilution and/or the fixing of the aroma chemical(s), i.e. the compounds of formula (I) and optionally one or more further aroma chemical different from compounds (I), as defined above, comprised in the aroma chemical composition.

Suitable carrier materials can be liquid or oil-like carrier materials as well as wax-like or solid carrier materials.

Suitable liquid or oil-like carrier materials are selected, for example, from water, alcohols, such as methanol or ethanol, aliphatic diols and polyols with a melting temperature below 20° C, such as ethylene glycol, glycerol, diglycerol, propylene glycol or dipropylene glycol and 1,2-butylene glycol, cyclic siloxanes, such as hexamethylcyclotrisiloxane or decamethylcyclopentasiloxane, diethylene glycol monoethyl ether, diethyl phthalate, isopropyl myristate, triethyl citrate, benzyl benzoate, vegetable oils, such as fractionated coconut oil or esters of fatty alcohols with melting temperatures below 20° C, such as tetradecyl acetate or tetradecyl lactate, esters of glycerol with melting temperatures below 20° C, and alkyl esters of fatty acids with melting temperatures below 20° C, such as isopropyl myristate.

Suitable wax-like or solid carrier materials are selected, for example, from fatty alcohols with melting temperatures below 20° C, such as myristyl alcohol, stearyl alcohol or cetyl alcohol, polyols with melting temperatures above 20° C, fatty acid esters with fatty alcohols which have a melting temperature of above 20° C, such as lanolin, beeswax, carnauba wax, candelilla wax or Japan wax, waxes produced from petroleum, such as hard paraffin, water-insoluble porous minerals, such as silica gel, silicates, for example talc, microporous crystalline aluminosilicates (zeolites), clay minerals, for example bentonite, or phosphates, for example sodium tripolyphosphate, paper, cardboard, wood, textile composite or nonwoven materials made of natural and/or synthetic fibers.

Suitable carrier materials are also selected, for example, from water-soluble polymers, such as polyacrylic acid esters or quaternized polyvinylpyrrolidones, or water-alcohol-soluble polymers, such as specific thermoplastic polyesters and polyamides. The polymeric carrier material can be present in various forms, e.g. in the form of a gel, a paste, solid particles, such as microcapsules, or brittle coatings.

Preferably, the aroma chemical composition is selected from fragranced ready-to-use compositions, as defined above.

Generally, the total amount of the at least one compound of formula (I), a stereoisomer thereof or a mixture of stereoisomers thereof in the aroma chemical compositions according to the present invention are typically adapted to the particular intended use or the intended application and can, thus, vary over a wide range. As a rule, the customary standard commercial use amounts for scents are used.

Accordingly, the total amount of the at least one compound of formula (I), a stereoisomer thereof or a mixture of stereoisomers thereof in the composition is in the range of from 0.001 to 99.9% by weight, preferably in the range of from 0.01 to 90% by weight, more preferably in the range of from 0.05 to 80% by weight, even more preferably in the range of from 0.1 to 60% by weight, in particular in the range of from 0.1 to 40% by weight, based on the total weight of the composition.

In one embodiment of the invention, the total amount of the at least one compound of formula (I), a stereoisomer thereof or a mixture of stereoisomers thereof in the composition is in the range of from 0.001 to 5 weight%, preferably from 0.01 to 2 weight%, based on the total weight of the composition.

A further embodiment of the invention is directed to a composition comprising at least one compound of formula (I), a stereoisomer thereof or a mixture of stereoisomers thereof and at least one component selected from the group consisting of surfactants, emollients and solvents.

One embodiment of the invention is directed to a composition comprising at least one compound of formula (I), a stereoisomer thereof or a mixture of stereoisomers thereof and at least one solvent.

In the context of the present invention, a "solvent" serves for the dilution of the compound of formula (I), a stereoisomer thereof or a mixture of stereoisomers thereof, as defined above. Some solvents have fixing properties at the same time.

The one or more solvent(s) can be present in the composition from 0.01 to 99% by weight based on the composition. In a preferred embodiment of the invention, the composition comprise 0.1 to 90% by weight, preferably 0.5 to 80% by weight of solvent(s) based on the composition. The amount of solvent(s) can be chosen depending on the composition. In one embodiment of the invention, the composition comprises 0.05 to 10% by weight, preferably 0.1 to 5% by weight, more preferably 0.2 to 3% by weight based on the composition. In one embodiment of the invention, the composition comprises 20 to 70% by weight, preferably 25 to 50% by weight of solvent(s) based on the composition.

Preferred solvents are ethanol, dipropylene glycol (DPG), propylene glycol, 1,2-butylene glycol, glycerol, diethylene glycol monoethyl ether, diethyl phthalate (DEP), isopropyl myristate (IPM), triethyl citrate (TEC), and benzyl benzoate (BB).

Especially preferred solvents are selected from the group consisting of ethanol, propylene glycol, dipropylene glycol, triethyl citrate, benzyl benzoate and isopropyl myristate.

In a preferred embodiment of the invention, the solvent is selected from the group consisting of ethanol, isopropanol, diethylene glycol monoethyl ether, glycerol, propylene glycol, 1,2-butylene glycol, dipropylene glycol, triethyl citrate and isopropyl myristate.

According to a further aspect, the compounds of formula (I), the stereoisomers thereof or mixtures of stereoisomers thereof are suitable for use in surfactant-containing compositions. According to their characteristic scent profiles, the compounds of formula (I), the stereoisomers thereof or mixtures of stereoisomers thereof can especially be used for the perfuming of surfactant-containing compositions such as, for example, cleaners (in particular laundry cleaners and all-purpose cleaners).

One embodiment of the invention is therefore directed to a composition comprising at least one compound of formula (I), a stereoisomer thereof or a mixture of stereoisomers thereof and at least one surfactant.

The surfactant(s) may be selected from anionic, non-ionic, cationic and/or amphoteric or zwitterionic surfactants. Surfactant-containing compositions, such as for example shower gels, foam baths, shampoos, etc., preferably contain at least one anionic surfactant.

The compositions according to the invention usually contain the surfactant(s), in the aggregate, in a quantity of 0 to 40% by weight, preferably 0 to 20% by weight, more preferably 0.1 to 15% by weight, and particularly 0.1 to 10% by weight, based on the total weight of the composition. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolyzates (particularly wheat-based vegetable products), polyol fatty acid esters, sugar esters, sorbitan esters, polysorbates and amine oxides. If the nonionic surfactants contain polyglycol ether chains, they may have a conventional homolog distribution, although they preferably have a narrow-range homolog distribution.

Zwitterionic surfactants are surface-active compounds which contain at least one quaternary ammonium group and at least one -COO⁽⁻⁾ or -SO₃⁽⁻⁾ group in the molecule. Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, cocoacylaminopropyl dimethyl ammonium glycinate, and 2-alkyl-3-carboxymethyl-3-hydroxyethyl imidazolines, containing 8 to 18 carbon atoms in the alkyl or acyl group, and cocoacylaminoethyl hydroxyethyl carboxymethyl glycinate. The fatty acid amide derivative known under the CTFA name of Cocamidopropyl Betaine is particularly preferred.

Ampholytic surfactants are also suitable, particularly as co-surfactants. Ampholytic surfactants are surface-active compounds which, in addition to a C8 to C18 alkyl or acyl group, contain at least one free amino group and at least one -COOH- or - SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalk-ylaminopropionate, cocoacylaminoethyl aminopropionate and acyl sarcosine.

Anionic surfactants are characterized by a water-solubilizing anionic group such as, for example, a carboxylate, sulfate, sulfonate or phosphate group and a lipophilic group. Dermatologically safe anionic surfactants are known to the practitioner in large numbers from relevant textbooks and are commercially available. They are, in particular, alkyl sulfates in the form of their alkali metal, ammonium or alkanolammonium salts, alkylether sulfates, alkylether carboxylates, acyl isethionates, acyl sarcosinates, acyl taurines containing linear C12 to C8 alkyl or acyl groups and sulfosuccinates and acyl glutamates in the form of their alkali metal or ammonium salts.

Particularly suitable cationic surfactants are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example, cetyl trimethyl ammonium chloride, stearyl trim ethyl ammonium chloride, distearyl dimethyl ammonium chloride, lauryl dimethyl ammonium chloride, lauryl dimethyl benzyl ammonium chloride and tricetyl methyl ammonium chloride. In addition, the readily biodegradable quaternary ester compounds, such as, for example, the dialkyl ammonium methosulfates and methyl hydroxyalkyl dialkoyloxyalkyl ammonium methosulfates marketed under the name of Stepantexe and the corresponding products of the Dehyquart^{®} series, may be used as cationic surfactants. "Esterquats" are generally understood to be quaternized fatty acid triethanolamine ester salts. They can provide the compositions with particular softness. They are known substances which are prepared by the relevant methods of organic chemistry. Other cationic surfactants suitable for use in accordance with the invention are the quaternized protein hydrolyzates.

One embodiment of the invention is directed to a composition comprising at least one compound of formula (I), a stereoisomer thereof or a mixture of stereoisomers thereof and at least one oil component.

The oil components are typically present in a total quantity of 0.1 to 80, preferably 0.5 to 70, more preferably 1 to 60, even more preferably 1 to 50% by weight, in particular 1 to 40% by weight, more particularly 5 to 25% by weight and specifically 5 to 15% by weight based on the composition.

The oil components may be selected, for example, from Guerbet alcohols based on fatty alcohols con taining 6 to 18 and preferably 8 to 10 carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of C₁₈-C₃₈-alkyl-hydroxycarboxylic acids with linear or branched C₆-C₂₂-fatty alcohols, more especially dioctyl malate, esters of linear and/or branched fatty acids with polyhydric alcohols (for example propylene glycol, dimer dial or trimer triol), triglycerides based on C₆-C₁₀-fatty acids, liquid mono-, di- and triglyceride mixtures based on C₆-C₁₈-fatty acids, esters of C₆-C₂₂-fatty alcohols and/or Guerbet alcohols with aromatic carboxylic acids, more particularly benzoic acid, esters of dicarboxylic acids with polyols containing 2 to 10 car- bon atoms and 2 to 6 hydroxyl groups, vegetable oils, branched primary alcohols, substituted cyclohexanes, linear and branched C₆-C₂₂-fatty alcohol carbonates such as, for example, dicaprylyl carbonate (Cetiol@ CC), Guerbet carbonates based on fatty alcohols containing 6 to 18 and preferably 8 to 10 carbon atoms, esters of benzoic acid with linear and/or branched C₆ to C₂₂-alcohols (for example Finsolv^{®} TN), linear or branched, symmetrical or nonsymmetrical dialkyl ethers containing 6 to 22 carbon atoms per alkyl group such as, for example, dicaprylyl ether (Cetiol^{®} OE), ring opening products of epoxidized fatty acid esters with polyols and hydrocarbons or mixtures thereof.

### Method of preparation:

A further embodiment of the present invention relates to A method of preparing a fragranced ready-to-use composition, comprising incorporating at least one compound of formula (I), a stereoisomer thereof or a mixture of stereoisomers thereof, as defined above, into a ready-to-use composition.

This method comprises the incorporation of at least one compound of formula (I), a stereoisomer thereof or a mixture of stereoisomers thereof, to an ready-to-use composition, which has no or no notable sensory properties, on order to provide a specific odor and/or a specific flavor to this ready-to-use composition. In addition, this method also comprises the modification of the odor and/or the flavor of an ready-to-use composition, which already has notable sensory properties, by incorporating at least one compound of formula (I), a stereoisomer thereof or a mixture of stereoisomers thereof, into said ready-to-use composition.

Preferred ready-to-use compositions are those mentioned above.

The total amount of the at least one compound of formula (I), a stereoisomer thereof or a mixture of stereoisomers thereof, that is incorporated in the ready-to-use compositions highly depend on the intended use or the intended application and can, thus, vary over a wide range. Typical amounts of the at least one compound of formulae (I), a stereoisomer thereof or a mixture of stereoisomers thereof, that is incorporated in the ready-to-use compositions are those as defined above for the compositions.

The compounds of formula (I), the stereoisomers thereof or a mixture of stereoisomers thereof used according to the invention, the compositions obtainable by the above method of the invention, as well as the aroma chemical compositions according to the invention comprising them can also be in microencapsulated form, spray-dried form, in the form of inclusion complexes or in the form of extrusion products. The properties can be further optimized by so-called "coating" with suitable materials with regard to a more targeted release of the scent, for which purpose preferably waxy synthetic substances such as e.g. polyvinyl alcohol are used.

The microencapsulation can take place for example by the so-called coacervation method with the help of capsule materials, e.g. made of polyurethane-like substances or soft gelatin. The spray-dried perfume oils can be produced for example by spray-drying an emulsion or dispersion comprising the compounds of formula (I), the stereoisomers thereof or a mixture of stereoisomers thereof, wherein carrier substances that can be used are modified starches, proteins, dextrin and vegetable gums. Inclusion complexes can be prepared e.g. by introducing dispersions of fragrance compositions and cyclodextrins or urea derivatives into a suitable solvent, e.g. water. Extrusion products can be produced by melting the compounds of formula (I), the stereoisomers thereof or a mixture of stereoisomers thereof with a suitable wax-like substance and by extrusion with subsequent solidification, optionally in a suitable solvent, e.g. isopropanol.

A further embodiment of the present invention relates to a compound of the general formula (I.a) 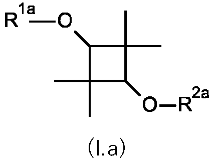 wherein
- R^{1a}: is C₂-C₄-alkyl and
- R^{2a}: is hydrogen or C₂-C₄-alkyl,
a stereoisomer thereof or a mixture of stereoisomers thereof.

A preferred embodiment of the present invention relates to the di-ether compounds of the general formula (I.a), i.e. to compounds of the general formula (I.a), wherein R^{1a} and R^{2a}, independently of one another, are selected from C₂-C₄-alkyl.

Further preferred are di-ether compounds of the general formula (I.a), wherein the radicals R^{1a} and R^{2a} are identical.

Examples of preferred compounds of the general formula (I.a) are
2,4-diethoxy-1,1,3,3-tetramethyl-cyclobutane,
2,4-diethoxy-1,1,3,3-tetramethyl-cyclobutane, which is essentially present **in** the form of its cis isomer,
2,4-diethoxy-1,1,3,3-tetramethyl-cyclobutane, which is essentially present **in** the form of its trans isomer,
2,4-di-n-propoxy-1,1,3,3-tetramethyl-cyclobutane,
2,4-di-n-propoxy-1,1,3,3-tetramethyl-cyclobutane, which is essentially present **in** the form of its cis isomer,
2,4-di-n-propoxy-1,1,3,3-tetramethyl-cyclobutane, which is essentially present **in** the form of its trans isomer,
2,4-diisopropoxy-1,1,3,3-tetramethyl-cyclobutane,
2,4-diisopropoxy-1,1,3,3-tetramethyl-cyclobutane, which is essentially present **in** the form of its cis isomer,
2,4-diisopropoxy-1,1,3,3-tetramethyl-cyclobutane, which is essentially present **in** the form of its trans isomer,
2,4-di-n-butoxy-1,1,3,3-tetramethyl-cyclobutane,
2,4-di-n-butoxy-1,1,3,3-tetramethyl-cyclobutane, which is essentially present **in** the form of its cis isomer,
2,4-di-n-butoxy-1,1,3,3-tetramethyl-cyclobutane, which is essentially present **in** the form of its trans isomer,
2,4-diisobutoxy-1,1,3,3-tetramethyl-cyclobutane,
2,4-diisobutoxy-1,1,3,3-tetramethyl-cyclobutane, which is essentially present **in** the form of its cis isomer, and
2,4-diisobutoxy-1,1,3,3-tetramethyl-cyclobutane, which is essentially present **in** the form of its trans isomer.

More preferred are compounds of the general formula (I.a), wherein the radicals R^{1a} and R^{2a} are selected from the group consisting of ethyl, n-propyl and ispopropyl.

Even more preferred are compounds of the general formula (I.a), wherein the radicals R^{1a} and R^{2a} are identical and selected from the group consisting of ethyl, n-propyl and ispopropyl.

Particularly preferred compounds (1.a) are
2,4-diethoxy-1,1,3,3-tetramethyl-cyclobutane,
2,4-diethoxy-1,1,3,3-tetramethyl-cyclobutane, which is essentially present in the form of its cis isomer, and
2,4-diethoxy-1,1,3,3-tetramethyl-cyclobutane, which is essentially present in the form of its trans isomer.

The compounds of the general formula (I.a) can be present in the form of a cis/trans isomer mixture, or can be essentially present in the form of their cis isomers or their trans isomers, respectively.

Regarding the expressions "cis/trans isomer mixture" and "essentially present", reference is made to the statements given above for the compounds of the general formula (I).

Preferably, the compounds of the general formula (I.a) are present in the form of a cis/trans isomer mixture.

The compounds of the general formula (I.a) can be prepared as described for the compounds of the general formula (I).

Accordingly, the present invention also relates to a method for preparing the compound of formula (I.a), a stereoisomer thereof or a mixture of stereoisomers thereof, as defined above, comprising
(i) reacting 2,2,4,4-tetramethylcyclobutane-1,3-diol with the alkylation reagent R^{1a}-X and optionally with the alkylation reagent R^{2a}-X, wherein R^{1a} and R^{2a} have one of the meanings given above and X represents a leaving group, selected from halogen, such as Cl, Br, I, and sulfonates, such as tosylate, mesylate, triflate or nonaflate, in the presence of a base to obtain a raw-product mixture, and
(ii) subjecting the raw-product mixture obtained in step (i) to a purification step.

The alkylation reaction in step (i) is performed under conventional alkylation reaction conditions that are well known to the skilled person.

Suitable bases that can be used in the alkylation reaction are as defined above.

For the preparation of the mono-ether compounds of the general formula (I.a), 2,2,4,4-tetramethylcyclobutane-1,3-diol is typically reacted with sub-equimolar or almost equimolar amounts, e.g. 0.7, 0.8, 0.9, or 0.95 equivalents, of the alkylation reagent R^{1a}-X to give the mono-substituted compound (I.a-OH), 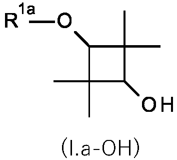
wherein R^{1a} has one of the meanings given above,
which is further subjected to a purification step, as defined above, in order to remove unwanted by-products or impurities, such as residual starting material or the corresponding di-substituted compounds.

The preparation of di-ether compounds of the general formula (I.a), where the radicals R^{1a} and R^{2a} are identical, is typically performed by reacting 2,2,4,4-tetramethylcyclobutane-1,3-diol with at least two equivalents, e.g. 2.0, 2.1, 2.5 or 3.0 equivalents, of the alkylation reagent R^{1a}-X or R^{2a}-X, respectively, wherein the radicals R^{1a}, R^{2a} and X have one of the meanings given above, and the obtained raw product is subsequently subjected to a purification step, as defined above.

The preparation of di-ether compounds of the general formula (I), where the radicals R^{1a} and R^{2a} are different, is generally performed by first reacting 2,2,4,4-tetramethylcyclobutane-1,3-diol with sub-equimolar or almost equimolar amounts, e.g. 0.7, 0.8, 0.9, or 0.95 equivalents, of the alkylation reagent R¹-X to give the mono-substituted compound (I.-a-OH), which after a purification step is further reacted with the second alkylation reagent R^{2a}-X.

Generally, the starting material 2,2,4,4-tetramethylcyclobutane-1,3-diol is applied as cis/trans isomer mixture. Consequently, the compounds I.a are typically obtained as a mixture of cis/trans isomers.

In step (ii) of the method for preparing the compound of formula (I.a), the raw-product mixture obtained in step (i) is subjected to a purification step, in order to remove unwanted by-products or impurities, such as residual mono-substituted compound (I.a-OH) and, if desired, to separate the cis/trans isomers.

Generally the purification in step (ii) can be performed by using common purification methods, such as crystallization, distillation or chromatographic methods, e.g. column chromatography or high performance liquid chromatography. In case where only minor amounts of impurities and by-products are present in the raw-product mixture the purification step can also be performed via a simple extractive workup. If desired the cis/trans isomers can be separated by fractional crystallization, column chromatography or high performance liquid chromatography. Alternatively, the cis/trans isomers can also be separated by using the method as described in US 3,227,764.

A further embodiment of the present invention relates to a compound of the general formula (I.b) 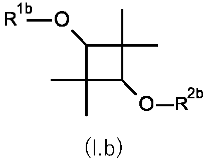 wherein
R^{1b} is -(C=O)-R³,
R^{2b} is hydrogen or -(C=O)-R⁴ and
R³ and R⁴, independently of one another, are selected from the group consisting of hydrogen and C₁-C₄-alkyl, with the provision that R³ and R⁴, if present, are different, a stereoisomer thereof or a mixture of stereoisomers thereof;
except for 2,2,4,4-tetramethylcyclobutanediol monobutyrate.

A preferred embodiment of the present invention relates to the di-ether compounds of the general formula (I.b), i.e. to compounds of the general formula (I.b), wherein R^{2b} is -(C=O)-R⁴.

Further preferred are compounds of the general formula (I.b), where R³ and R⁴ are selected from the group consisting of hydrogen and C₁-C₃-alkyl, in particular from the group consisting of hydrogen, methyl and ethyl.

The compounds of the general formula (I.b) can be prepared as described for the ester compounds of the general formula (I).

Accordingly, the present invention also relates to a method for preparing the compound of formula (I.b), a stereoisomer thereof or a mixture of stereoisomers thereof, as defined above, comprising
(i) reacting 2,2,4,4-tetramethylcyclobutane-1,3-diol with the carboxylic acid R³-COOH or an anhydride thereof, or with the acid halogenide R³-(C=O)X' in the presence of an organic base and optionally with the carboxylic acid R⁴-COOH or an anhydride thereof, or with the acid halogenide R⁴-(C=O)X', wherein R³ and R⁴ have one of the meanings given above and X' represents a halogen, such as Cl, Br or I, to obtain a raw-product mixture, and
(ii) subjecting the raw-product mixture obtained in step (i) to a purification step.

### EXAMPLES

### Analytics:

The purity of the products was determined by Gas Chromatography area-%:
For the esters:
   GC-system: Agilent 7890 B;
   GC-Column: CP-SIL 13 (50 m (Length), 0.32 mm (ID), 1.2 micrometer (film));
   Temperature program: 120° C to 200° C at 3° /min, 200° C to 250° C at 20° /min.
For the ethers:
   GC-system: Agilent 6890 N;
   GC-Column: DB1 (30 m (Length), 0.25 mm (ID), 0.25 micrometer (film));
   Temperature program: Injection at 10° C, 50° C to 120° C at 3° /min, 120° C to 200° C at 20° /min.

### 1. Preparation examples

### 1.1 Preparation of (3-formyloxy-2,2,4,4-tetramethyl-cyclobutyl) formate

| **Compound** | **MW** | **Mass/Volume** | **Moles** |
|---|---|---|---|
| 2,2,4,4-tetramethyl-cyclobutane-1,3-diol | 144.21 | 10g | 0.069 |
| formic acid | 46.03 | 22.3g | 0.485 |
| acetic anhydride | 102.09 | 42.5g | 0.416 |
| N,N'-dimethylaminopyridine | 122.17 | 0.17g | 0.001 |
| tetrahydrofuran | | 70mL | |

At 0° C formic acid was slowly added to acetic anhydride. The mixture was stirred 2 h at 55° C. The mixture was then cooled down to 0° C and 20 mL THF were added, at this temperature a solution of the alcohol in 50 mL THF was slowly added. After the addition DMAP was added to the mixture. The reaction was stirred at room temperature for 3.5 h and full conversion was observed by GC. 60 mL toluene was added to the mixture and the organic phase was washed 3 times with 30 mL of water. The organic phase was dried with sodium sulfate and the solvent was removed in the rotatory evaporator to give 9.7 g of crude product that contained 96% of the di-formate according to the GC (area %). The crude product was purified by silica gel chromatography. The corresponding di-formate was obtained with a purity of 99.2% (GC area %). The NMR indicated the presence of the product as cis:trans isomers in a ratio 55:45.

Characterization of the product:
GC: 99.2% purity (GC area %)
¹³C-NMR:

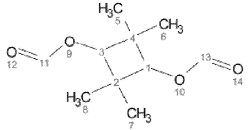

| Atom number | ¹³C shifts measured (multiplicity) | ¹³C shifts calculated (multiplicity) |
|---|---|---|
| C1/ C3 | 80.42 / 79.50 (d) | 80.30 (d) |
| C2/ C4 | 41.08 / 39.57 (s) | 39.45 (s) |
| C5/ C6/ C7/ C8 | 28.33 (cis) / 22.67 (trans)/ 16.86 (cis) (q) | 22.55 (q) |
| C11/ C13 | 160.62 (d) | 160.48 (d) |

### 1.2 Preparation of (3-acetoxy-2,2,4,4-tetramethyl-cyclobutyl) acetate

| **Compound** | **MW** | **Mass/Volum e** | **Moles** |
|---|---|---|---|
| 2,2,4,4-tetramethyl-cyclobutane-1,3-diol | 144.21 | 75 g | 0.520 |
| acetic anhydride | 102.09 | 146 g | 1.430 |
| N,N'-dimethylaminopyridine | 122.17 | 1.906 g | 0.016 |
| tetrahydrofuran | | 350 mL | |

DMAP was added to the THF solution of the diol at RF. The mixture was set to reflux (53° C) and acetic anhydride (2.25 eq) was slowly added at this temperature. After 2 h, 99% diol conversion was observed. However, ca. 15% of the mono-acetate was found in the mixture, for this reason, 0.5eq of acetic anhydride were added to the reaction. After 3 h at reflux 97% conversion to the diacetate was observed by GC. The reaction was cooled down to room temperature and slowly quenched with 150 mL of water, 300 mL of ethyl acetate and 200 mL of water were added. The organic phase was separated and washed with NaHCO₃ and brine solution. The organic extracts were combined and dried with sodium sulfate. After evaporating the solvent at reduced pressure, 102.2 g of crude product that contained > 97% of the diacetate according to the GC (area %) were obtained. The product was purified by distillation. The corresponding di-acetate was obtained with a purity of 99.6% (GC area %). The NMR indicated the presence of the product as cis:trans isomers in a ratio 55:45.

Characterization of the product:
GC: 99.6% purity (GC area %)
¹³C-NMR:

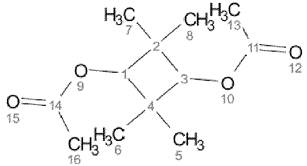

| Atom number | ¹³C shifts measured (multiplicity) | ¹³C shifts calculated (multiplicity) |
|---|---|---|
| C1/ C3 | 80.62/ 79.69 (d) | 80.54 (d) |
| C2/ C4 | 41.11/ 39.68 (s) | 39.60 (s) |
| C5/ C6/ C7/ C8 | 28.55 (cis)/ 22.61 (trans)/ 16.61 (cis) (q) | 22.56 (q) |
| C11/ C14 | 170.83/ 170.81 (d) | 170.72 (s) |
| C13/ C16 | 20.69/ 20.59 (q) | 20.61 (q) |

### 1.3 Preparation of (3-propanoyloxy-2,2,4,4-tetramethyl-cyclobutyl) propanoate

| **Compound** | **MW** | **Mass/Volume** | **Moles** |
|---|---|---|---|
| 2,2,4,4-tetramethyl-cyclobutane-1,3-diol | 144.21 | 8 g | 0.055 |
| propionyl chloride | 92.52 | 12.83 g | 0.139 |
| N,N'-dimethylaminopyridine | 122.17 | 0.120 g | 0.001 |
| triethylamine | 101.19 | 14 g | 0.139 |
| hexane | | 100 mL | |

A solution of the alcohol, triethylamine and DMAP in hexane (50 mL) was cooled down to 0° C. At this temperature, propionyl chloride was slowly added. The reaction was stirred at room temperature for 1 h, 50 mL of hexane were added and the reaction was stirred 1 h at reflux (67° C). After this time full conversion was observed by GC. The reaction was cooled down to room temperature and slowly quenched with 30 mL of water, 50 mL of hexane were added and the organic phase was washed with NaHCO₃ and brine solution. The organic extracts were combined and dried with sodium sulfate. After evaporating the solvent at reduced pressure, 13 g of crude product that contained 64% of the dipropionate according to the GC (area %) and 25% of the mono-propionate (GC area%) were obtained. The product was purified by silica gel chromatography. The corresponding di-propionate was obtained with a purity of 94% (GC area %). The NMR indicated the presence of the product as cis:trans isomers in a ratio 1:1.

Characterization of the product:
GC: 94% purity (GC area %)
¹³C-NMR:

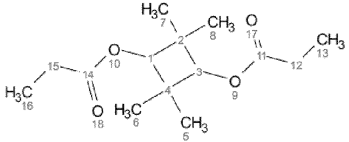

| Atom number | ¹³C shifts measured (multiplicity) | ¹³C shifts calculated (multiplicity) |
|---|---|---|
| C1/ C3 | 80.44 / 79.56 (d) | 80.39 (d) |
| C2/ C4 | 41.18 / 39.79 (s) | 39.75 (s) |
| C5/ C6/ C7/ C8 | 28.54 (cis) / 22.60 (trans) / 16.59 (cis) (q) | 22.56 (q) |
| C11/ C14 | 174.25 / 174.23 (d) | 174.19 (s) |
| C13/ C16 | 9.28 (q) | 9.24 (q) |
| C12/ C15 | 27.52 / 27.42 (t) | 27.48 (t) |

### 1.4 Preparation of [3-(2,2-dimethylpropanoyloxy)-2,2,4,4-tetramethyl-cyclobutyl] 2,2-dimethylpropanoate

| **Compound** | **MW** | **Mass/Volume** | **Moles** |
|---|---|---|---|
| 2,2,4,4-tetramethyl-cyclobutane-1,3-diol | 144.21 | 50 g | 0.347 |
| Pivaloyl chloride | 120.58 | 156.754 g | 1.3 |
| N,N'-dimethylaminopyridine | 122.17 | 2.118 g | 0.017 |
| triethylamine | 101.19 | 87.710 g | 0.867 |
| THF | | 250 mL | |

A solution of the alcohol, triethylamine and DMAP in THF was cooled down to 0° C. At this temperature, pivaloyl chloride (2.5) was slowly added. The reaction was stirred at room temperature for 0.5 h, and 4 h at reflux. After this time, 96% conversion was observed, however only 49% of the dipivalate was detected by GC being the rest the mono-derivative. Accordingly, 1.25eq of pivaloyl chloride were further added and the reaction was stirred at RF for 21 h. The reaction was cooled down to room temperature and slowly quenched with 200 mL of water, 200 mL of ethyl acetate were added and the organic phase was washed with NaHCO₃ and brine solution. The organic extracts were combined and dried with sodium sulfate. After evaporating the solvent at reduced pressure, 110.8 g of crude product that contained 88% of the dipivalate according to the GC (area %) and 11% of the mono-pivalate (GC area%) were obtained. The product was purified by distillation. The corresponding di-pivalate was obtained with a purity of 99.8% (GC area %). The NMR indicated the presence of the product as cis:trans isomers in a ratio 55:45.

Characterization of the product:
GC: 99.8% purity (GC area %)
¹³C-NMR:

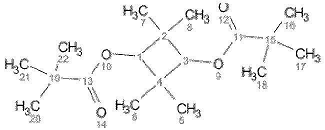

| Atom number | ¹³C shifts measured (multiplicity) | ¹³C shifts calculated (multiplicity) |
|---|---|---|
| C1/ C3 | 80.31 / 79.49 (d) | 79.48 (d) |
| C2/ C4 | 41.35 / 40.09 (s) | 41.35 (s) |
| C5/ C6 / C7 / C8 | 28.45 (cis) / 22.59 (trans) / 16.61 (cis) (q) | 28.44 (q) / 16.61 (q) |
| C11/ C13 | 178.22 / 178.21 (s) | 178.30 (s) |
| C15/ C19 | 39.07 / 39.06 (s) | 39.08 (s) |
| C16/ C17/ C18/ C20/ C21/ C22 | 27.29 (q) | 27.28 (q) |

### 1.5 Preparation of [3-(2-methylpropanoyloxy)-2,2,4,4-tetramethyl-cyclobutyl] 2-methylpropanoate

| **Compound** | **MW** | **Mass/Volume** | **Moles** |
|---|---|---|---|
| 2,2,4,4-tetramethyl-cyclobutane-1,3-diol | 144.21 | 10 g | 0.069 |
| Isobutyric anhydride | 158.19 | 24.68 g | 0.156 |
| N,N'-dimethylaminopyridine | 122.17 | 0.254 g | 0.03 |
| tetrahydrofuran | | 50 mL | |

DMAP was added to the THF solution of the diol at RF. The mixture was set to reflux (60° C) and isobutyric anhydride (2.25eq) was slowly added at this temperature. After 2.5 h, 99% diol conversion was observed. However, ca. 8% of the mono-isobutyrate was found in the mixture. The reaction was cooled down to room temperature and slowly quenched with 20 mL of water, 50 mL of ethyl acetate. The organic phase was separated and washed with NaHCO₃ and brine solution. The organic extracts were combined and dried with sodium sulfate. After evaporating the solvent at reduced pressure, 19.6 g of crude product that contained > 91% of the di-isobutyrate according to the GC (area %) were obtained. The product was purified by distillation. The product was purified by distillation. The corresponding di-isobutyrate was obtained with a purity of 97.4% (GC area %). The NMR indicated the presence of the product as cis:trans isomers in a ratio 55:45.

Characterization of the product:
GC: 97.4% purity (GC area %)
¹³C-NMR:

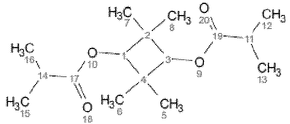

| Atom number | ¹³C shifts measured (multiplicity) | ¹³C shifts calculated (multiplicity) |
|---|---|---|
| C1/ C3 | 80.35 / 79.52 (d) | 79.48 (d) |
| C2/ C4 | 41.25 / 39.93 (s) | 41.21 (s) |
| C5/ C6 / C7/C8 | 28.51 (cis) / 22.59 (trans) / 16.58 (cis) (q) | 28.47 (q) / 16.54 (q) |
| C19/ C17 | 176.97 / 176.94 (s) | 176.92 (s) |
| C11/ C14 | 34.17 / 34.11 (d) | 34.07 (d) |
| C12/ C13/ C15/ C16 | 19.10 / 18.79 (q) | 19.06 (q) |

### 1.6 Preparation of (3-butanoyloxy-2,2,4,4-tetramethyl-cyclobutyl) butanoate

| **Compound** | **MW** | **Mass/Volume** | **Moles** |
|---|---|---|---|
| 2,2,4,4-tetramethyl-cyclobutane-1,3-diol | 144.21 | 10 g | 0.069 |
| n-butyric anhydride | 158.19 | 24.68 g | 0.156 |
| N,N'-dimethylaminopyridine | 122.17 | 0.254 g | 0.03 |
| Tetrahydrofuran | | 50 mL | |

DMAP was added to the THF solution of the diol at RF. The mixture was set to reflux (60° C) and n-butyric anhydride (2.25eq) was slowly added at this temperature. After 2.5 h, 99% diol conversion was observed. However, ca. 10% of the mono-n-butyrate was found in the mixture. The reaction was cooled down to room temperature and slowly quenched with 20 mL of water, 50 mL of ethyl acetate. The organic phase was separated and washed with NaHCO₃ and brine solution. The organic extracts were combined and dried with sodium sulfate. After evaporating the solvent at reduced pressure, 18.8 g of crude product that contained > 88% of the di-n-butyrate according to the GC (area %) were obtained. The product was purified by distillation. The product was purified by distillation. The corresponding di-n-butyrate was obtained with a purity of 99.7% (GC area %). The NMR indicated the presence of the product as cis:trans isomers in a ratio 55:45.

Characterization of the product:
GC: 99.7% purity (GC area %)
¹³C-NMR:

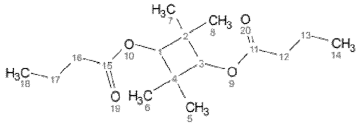

| Atom number | ¹³C shifts measured (multiplicity) | ¹³C shifts calculated (multiplicity) |
|---|---|---|
| C1/ C3 | 80.51/ 79.62 (d) | 79.55 (d) |
| C2/ C4 | 41.20/ 39.80 (s) | 41.14 (s) |
| C5/ C6/ C7/C8 | 28.54 (cis) / 22.68 (trans) / 16.74 (cis) (q) | 28.47 (q) |
| C11/ C15 | 173.62/ 173.60 (s) | 173.54 (s) |
| C12/ C16 | 36.13/ 36.04 (t) | 35.98 (t) |
| C13/ C17 | 18.50/ 18.21 (t) | 18.44 (t) |
| C14/ C18 | 13.76/ 13.62 (q) | 13.70 (q) |

### 1.7 Preparation of 2,4-dimethoxy-1,1,3,3-tetramethyl-cyclobutane

| **Compound** | **MW** | **Mass/Volume** | **Moles** |
|---|---|---|---|
| 2,2,4,4-tetramethyl-cyclobutane-1,3-diol | 144.21 | 30 g | 0.208 |
| Methyl iodide | 141.94 | 73.82 g | 0.520 |
| Sodium hydride | 23.99 | 12.476 g | 0.520 |
| Tetrahydrofuran | | 300 mL | |

To a dispersion of sodium hydride (2.5eq) in 150 mL of THF a solution of the caged diol in 150 mL of THF was slowly added at 0° C. The mixture was stirred 1 h at 0° C. After this time 2.5 eq. of methyl iodide were slowly added at RT. After the addition, the mixture was stirred at 40° C for 21 h. At this point, the reaction was slowly quenched with 150 mL of water. The organic phase was extracted with 3 times 150 mL MTBE. The organic extracts were combined and washed with 150 mL of NH₃ solution and with 150 mL brine solution. The organic extracts were combined and dried with sodium sulfate. After evaporating the solvent at reduced pressure 18.8 g of crude product were obtained with 54% of the di-methyl ether according to the GC (area %) being the rest the mono-methylated alcohol (30%) and the unreacted caged diol (10%). The di-methyl ether product was purified by silica gel chromatography. The corresponding di-methyl ether was obtained with a purity of 99.8% (GC area %). The NMR indicated the presence of the product as cis:trans isomers in a ratio 4:1.

Characterization of the product:
GC: 99.8% purity (GC area %)
¹³C-NMR:

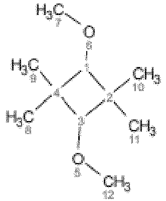

| Atom number | ¹³C shifts measured (multiplicity) | ¹³C shifts calculated (multiplicity) |
|---|---|---|
| C1/ C3 | 88.72/ 86.77 (d) | 86.76 (d) |
| C2/ C4 | 42.06/ 40.53 (s) | 42.05 (s) |
| C7/ C12 | 58.71/ 58.53 (q) | 58.32 (q) |
| C8/ C9/ C10/ C11 | 32.75 (cis) / 22.73 (trans) / 15.35 (cis) (q) | 15.35 (q) |

### 1.8 Preparation of 3-methoxy-2,2,4,4-tetramethyl-cyclobutanol

During the purification of the 2,4-dimethoxy-1,1,3,3-tetramethyl-cyclobutane in example 1.7, two isomers of the corresponding mono-methyl ether were isolated. 1.4 g of a highly trans isomer of 3-methoxy-2,2,4,4-tetramethyl-cyclobutanol (high trans) with a purity of 94% (94% trans, GC area %, RT: 20.70min) was obtained. The presence of a high fraction of the trans isomer was confirmed by NMR.

Characterization of the product (high trans):
GC: 94% purity (94% trans according to GC area %; RT: 20.70min)
¹³C-NMR:

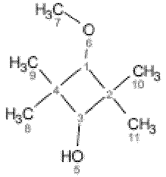

| Atom number | ¹³C shifts measured (multiplicity) | ¹³C shifts calculated (multiplicity) |
|---|---|---|
| C1 | 88.73 (d) | 88.75 (d) |
| C2/ C4 | 40.65 (s) | 40.64 (s) |
| C3 | 79.53 (d) | 79.50 (d) |
| C7 | 58.53 (q) | 58.52 (q) |
| C8/ C9 | 22.59 (q) | 22.60 (q) |
| C10/ C11 | 23.14 (q) | 23.17 (q) |

0.5 g of a highly cis isomer of 3-methoxy-2,2,4,4-tetramethyl-cyclobutanol was as well isolated with a purity of 98% (98% cis, GC area %, RT: 20.30 min). The presence of a highly cis isomer was confirmed by GC upon comparison with an cis/trans isomer mixture of 3-methoxy-2,2,4,4-tetramethyl-cyclobutanol.

Characterization of the product (high cis):
GC: 98% purity (98% cis according to GC area %; RT: 20.30min).

### 1.9 Preparation of 2,4-diethoxy-1,1,3,3-tetramethyl-cyclobutane

| **Compound** | **MW** | **Mass/Volume** | **Moles** |
|---|---|---|---|
| 2,2,4,4-tetramethyl-cyclobutane-1,3-diol | 144.21 | 20 g | 0.139 |
| Ethyl iodide | 155.97 | 82.25 g | 0.527 |
| Sodium hydride | 23.99 | 12.65 g | 0.527 |
| Tetrahydrofuran | | 200 mL | |

To a dispersion of 7.65 g of sodium hydride (2.3eq) in 100 mL of THF a solution of the caged diol in 150 mL of THF was slowly added at 0° C. The mixture was stirred 1 h at 0° C. After this time 2.3 eq. of ethyl iodide (49.75 g) were slowly added at RT. After the addition, the mixture was stirred at 40° C for 21 h. At this point, ca. 90% conversion of the alcohol was observed. However, only 35% of the diethyl ether was detected being the rest (56%) the mono-ether derivative. Accordingly, the reaction was cooled down to 0° C and 5 g of sodium hydride were added. After 15 min at this temperature, 32.5 g of ethyl iodide were slowly added. The mixture was stirred at 40° C during 22 h. The reaction was then slowly quenched with 100 mL of water. The organic phase was extracted with 3 times 100mL MTBE. The organic extracts were combined and washed with 100 mL of NH₃ solution and with 100mL brine solution. The organic extracts were combined and dried with sodium sulfate. After evaporating the solvent at reduced pressure 22.8 g of crude product were obtained with 61.5% of the di-ethyl ether according to the GC (area %) being the rest the mono-ethylated alcohol (29%) and the unreacted caged diol. The product was purified by silica gel chromatography. The corresponding di-ethyl ether was obtained with a purity of 99.8% (GC area %). The NMR indicated the presence of the product as cis:trans isomers in a ratio 3:1.

Characterization of the product:
GC:99.8% purity (GC area %)
¹³C-NMR:

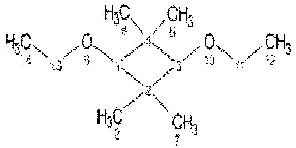

| Atom number | ¹³C shifts measured (multiplicity) | ¹³C shifts calculated (multiplicity) |
|---|---|---|
| C1/ C3 | 86.67/ 84.82 (d) | 84.81 (d) |
| C2/ C4 | 42.28/ 40.69 (s) | 42.28 (s) |
| C5/ C6/ C7/ C8 | 30.83 (cis)/ 23.57 (trans)/ 15.76 (cis) (q) | 30.82 (q) |
| C11/ C13 | 65.97/ 65.55 (t) | 65.54 (t) |
| C12/C14 | 15.30 (q) | 15.30 (q) |

### 1.10 Preparation of 3-ethoxy-2,2,4,4-tetramethyl-cyclobutanol

During the purification of 2,4-diethoxy-1,1,3,3-tetramethyl-cyclobutane in example 1.9, 2.9 g of the mono ethyl ether (3-ethoxy-2,2,4,4-tetramethyl-cyclobutanol) was isolated as well with a purity of 79% (GC area %), being the rest 20.5% the di-ethyl ether derivative (2,4-diethoxy-1,1,3,3-tetramethyl-cyclobutane). The NMR information indicated a trans:cis ratio of 55:45 for 3-ethoxy-2,2,4,4-tetramethyl-cyclobutanol and a cis:trans ratio of 3:1 for 2,4-diethoxy-1,1,3,3-tetramethyl-cyclobutane.

Characterization of the product:
GC: 79% purity (GC area %)
¹³C-NMR:

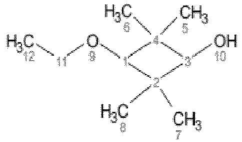

| Atom number | ¹³C shifts measured (multiplicity) |
|---|---|
| C1 | 86.59 / 84.87 (d) |
| C2/ C4 | 42.71 / 40.78 (s) |
| C3 | 78.33 (d) |
| C5/ C6/ C7/ C8 | 15.33 (cis)/ 23.97 (trans)/ 22.81 (trans)/ 29.81 (cis) (q) |
| C11 | 65.68 (t) |
| C12 | 15.29 (q) |

### 1.11 Preparation of 2,4-di-n-propoxy-1,1,3,3-tetramethyl-cyclobutane

| **Compound** | **MW** | **Mass/Volume** | **Moles** |
|---|---|---|---|
| 2,2,4,4-tetramethyl-cyclobutane-1,3-diol | 144.21 | 10 g | 0.069 |
| 1-lodopropan | 169.99 | 27.11 g | 0.159 |
| Sodium hydride | 23.99 | 3.83 g | 0.159 |
| Tetrahydrofuran | | 105 mL | |

To a dispersion of 3.83 g of sodium hydride (2.3eq) in 75 mL of THF a solution of the caged diol in 30 mL of THF was slowly added at 0° C. The mixture was stirred 30 min at 0° C. After this time 2.3 eq of 1-lodopropan (27.11g) were slowly added at RT. After the addition, the mixture was stirred at 50° C for 4 h. At this point, ca 80% conversion of the alcohol was observed. However, only 12% of the di-n-propyl ether was detected being the rest (ca. 65%) the mono-ether derivative. Accordingly, the reaction was cooled down to 0° C and 1.67 g of sodium hydride were added. After 30 min at this temperature, 11.8 g of 1-iodopropan were slowly added. The mixture was stirred at 50° C during 18 h. The reaction was then slowly quenched with 50 mL of water. The organic phase was extracted with 3 times 50 mL MTBE. The organic extracts were combined and washed with 50 mL of NH₃ solution and with 30 mL brine solution. The organic extracts were combined and dried with sodium sulfate. After evaporating the solvent at reduced pressure the crude product contained 22% of the di-n-propyl ether according to the GC (area %) being the rest the mono-n-propylated alcohol (67%) and the unreacted caged diol. The product was purified by silica gel chromatography. The corresponding di-n-propyl ether (0.7 g) was obtained with a purity of 99.4% (GC area %). The NMR indicated the presence of the product as cis:trans isomers in a ratio 85:15.

Characterization of the product:
GC: 99.4% purity (GC area %)
¹³C-NMR:

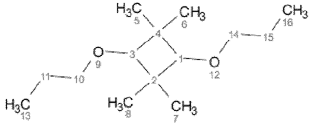

| Atom number | ¹³C shifts measured (multiplicity) |
|---|---|
| C1/ C3 | 84.99 (d) |
| C2/ C4 | 42.41 (s) |
| C5/ C8/ C6/ C7 | 15.73/ 14.14 (cis)/ 23.60/ 23.19 (trans)/ 30.91/ 29.74 (cis) (q) |
| C10/ C14 | 72.43/ 71.99 (t) |
| C11/ C15 | 23.19/ 23.60 (t) |
| C13/ C16 | 10.66 (q) |

### 1.12 Preparation of 3-propoxy-2,2,4,4-tetramethyl-cyclobutanol

During the purification of 2,4-di-n-propoxy-1,1,3,3-tetramethyl-cyclobutane in example 1.11, 0.6 g of the mono n-propyl ether (3-propoxy-2,2,4,4-tetramethyl-cyclobutanol) were isolated as well with a purity of 99% (GC area %). The NMR indicated a 3:2 trans:cis ratio.

Characterization of the product:
GC: 99% purity (GC area %)
¹³C-NMR:

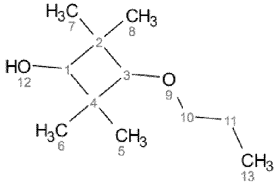

| Atom number | ¹³C shifts measured (multiplicity) |
|---|---|
| C1 | 79.80/ 78.44 (d) |
| C2/ C4 | 40.90/ 42.78 (s) |
| C3 | 86.69/ 84.97 (d) |
| C5/ C6/ C7/ C8 | 26.92 (cis)/ 29.85 (cis)/ 23.09 (trans)/ 22.83 (trans) / 15.29 (cis) (q) |
| C10 | 72.35/ 72.11 (t) |
| C11 | 23.21/ 23.16 (t) |
| C13 | 10.66 (q) |

### 1.13 Preparation of 2,4-di-n-butoxy-1,1,3,3-tetramethyl-cyclobutane

| **Compound** | **MW** | **Mass/Volume** | **Moles** |
|---|---|---|---|
| 2,2,4,4-tetramethyl-cyclobutane-1,3-diol | 144.21 | 10 g | 0.069 |
| 1-lodobutan | 184.02 | 29.35 g | 0.159 |
| Sodium hydride | 23.99 | 3.83 g | 0.159 |
| Tetrahydrofuran | | 105 mL | |

To a dispersion of 3.83 g of sodium hydride (2.3eq) in 75 mL of THF a solution of the caged diol in 30 mL of THF was slowly added at 0° C. The mixture was stirred 30 min at 0° C. After this time 2.3 eq of 1-lodobutan (29.35 g) were slowly added at RT. After the addition, the mixture was stirred at 50° C for 22 h. At this point, ca 75% conversion of the alcohol was observed. However, only 12% of the di-n-butyl ether was detected being the rest (ca. 65%) the mono-ether derivative according to the GC area%. The reaction was then slowly quenched with 50 mL of water. The organic phase was extracted with 3 times 50 mL MTBE. The organic extracts were combined and washed with 50 mL of NH₃ solution and with 30 mL brine solution. The organic extracts were combined and dried with sodium sulfate. After evaporating the solvent at reduced pressure, the crude product was purified by silica gel chromatography. The corresponding di-n-butyl ether (1.6 g) was obtained with a purity of 96% (GC area %). The NMR indicated the presence of the product as cis:trans isomers in a ratio 85:15.

Characterization of the product:
GC: 96% purity (GC area %)
¹³C-NMR:

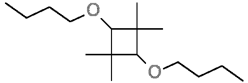

| Atom number | ¹³C shifts measured (multiplicity) |
|---|---|
| C1/ C3 | 84.99/ 86.84 (d) |
| C2/ C4 | 42.37/ 40.86 (s) |
| C5/ C8/ C6/ C7 | 15.72 (cis)/ 23.60 (trans)/ 30.91 (cis) (q) |
| C9/ C13 | 72.66/ 70.18 (t) |
| C10/ C14 | 32.10/ 32.18 (t) |
| C11/ C15 | 19.44/ 19.41 (t) |
| C12/ C16 | 14.04/ 13.97 (q) |

### 1.14 Preparation of 3-butoxy-2,2,4,4-tetramethyl-cyclobutanol

During the purification of 2,4-di-n-butoxy-1,1,3,3-tetramethyl-cyclobutane in example 1.13, 2g of the mono-n-butyl ether (3-butoxy-2,2,4,4-tetramethyl-cyclobutanol) were isolated as well with a purity of 99% (GC area %). The NMR indicated a 60:40 cis:trans ratio.

Characterization of the product:
GC: GC: 99% purity (GC area %)
¹³C-NMR:

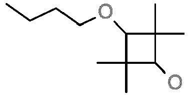

| Atom number | ¹³C shifts measured (multiplicity) |
|---|---|
| C1 | 79.74/ 78.37 (d) |
| C2/ C4 | 42.76/ 40.87 (s) |
| C3 | 86.74 (trans)/ 85.02 (cis) (d) |
| C5/ C6/ C7/ C8 | 29.87 (cis)/ 22.84 (trans)/ 23.13 (trans) / 15.33 (cis) (q) |
| C11 | 70.55/ 70.31(t) |
| C12 | 32.12/ 32.09 (t) |
| C13 | 19.44 (t) |
| C14 | 14.03 (q) |

### 1.15 Preparation of 2,4-di-tert.-butoxy-1,1,3,3-tetramethyl-cyclobutane

| **Compound** | **MW** | **Mass/Volume** | **Moles** |
|---|---|---|---|
| 2,2,4,4-tetramethyl-cyclobutane-1,3-diol | 144.21 | 10 g | 0.069 |
| Methyl tertbutyl ether | 88.15 | 140 mL | |
| Sulfuric acid | 98.08 | 13.6 g | 0.139 |
| Molecular sieve 4A | | 10 g | |

10 g of caged diol were dissolved in 140 mL of methyl tertbutyl ether (MTBE) and the 10 g of Molecular sieves 4A were added. To this dispersion the sulfuric acid was slowly added at 25° C. The reaction was stirred 6 h at 40° C. After this time, ca 90% diol conversion was observed. The molecular sieve was filtered and to the reaction 50 mL of NaHCO₃ were slowly added. After the phase separation, the aq. Phase was extracted twice with 30 mL MTBE. The organic extracts were combined, washed with 50 mL of of NaHCO₃ and dried with sodium sulfate. After evaporating the solvent at reduced pressure the crude product was purified by silica gel chromatography. The corresponding 2,4-di-tert.-butoxy-1,1,3,3-tetramethyl-cyclobutane (0.9 g) was obtained with a purity of 90% (GC area %). The NMR indicated the presence of the product as cis:trans isomers in a ratio 2:1.

Characterization of the product:
GC: GC: 90% purity (GC area %)
¹³C-NMR:

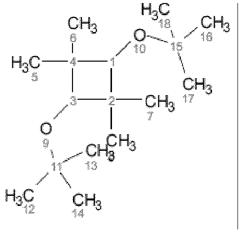

| Atom number | ¹³C shifts measured (multiplicity) |
|---|---|
| C1/ C3 | 78.27 (d) |
| C2/ C4 | 43.63/ 41.36 (s) |
| C5/ C6/ C7/ C8 | 29.83 (cis)/ 23.29 (trans) / 16.71 (cis) (q) |
| C11 | 71.69/ 71.35 (s) |
| C12/ C13/ C14/ C15/ C16/ C17/C18 | 28.74 (q) |

### 1.16 Preparation of 3-tert.-butoxy-2,2,4,4-tetramethyl-cyclobutanol

During the purification of 2,4-di-tert.-butoxy-1,1,3,3-tetramethyl-cyclobutane in example 1.15, 1.1 g of the mono tert.-butyl ether (3-tert.-butoxy-2,2,4,4-tetramethyl-cyclobutanol) was isolated as well with a purity of 90% (GC area %). The NMR indicated a 3:2 trans:cis ratio.

Characterization of the product:
GC: GC: 90% purity (GC area %)
¹³C-NMR:

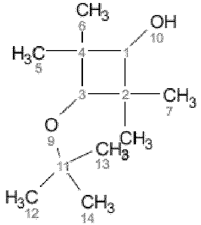

| Atom number | ¹³C shifts measured (multiplicity) |
|---|---|
| C1 | 79.72 (d) |
| C2/ C4 | 43.43/ 41.23 (s) |
| C3 | 78.52/ 78.23 (d) |
| C5/ C6/ C7/ C8 | 28.74 (cis)/ 23.31 / 22.22 (trans) / 15.77 (cis) (q) |
| C11 | 71.69 (t) |
| C12/ C13/ C14 | 28.45 (q) |

### 1.17 Preparation of (3-acetoxy-2,2,4,4-tetramethyl-cyclobutyl) acetate having a cis/trans isomer ratio of 9:1

68.5 g of the pure di-acetate sample prepared in example 1.2 were set in the fridge for a couple of minutes and it was observed how crystallization started. Accordingly, the sample was left in the freezer for 30 min. At this point the sample was completely solid. After this time, the sample was left at RT and it was observed how the crystals were slowly becoming liquid. A 10 mL fraction of this bi-phasic mixture was filtrated, and both the fractions were analyzed by GC. The liquid fraction contained a cis:trans mixture 52:48 (according to GC area %). The crystals, however were a 9:1 cis:trans mixture (according to GC area % and NMR).

Characterization of the product:
GC: 9:1 cis/trans isomer mixture
¹³C-NMR:

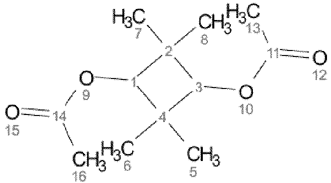

| Atom number | ¹³C shifts measured (multiplicity) | ¹³C shifts calculated (multiplicity) |
|---|---|---|
| C1/ C3 | 79.70 (d) | 80.54 (d) |
| C2/ C4 | 41.11 (s) | 39.60 (s) |
| C5/ C6/ C7/ C8 | 28.55 / 16.61 (q) | 22.53 (q) |
| C11/ C14 | 170.91 (d) | 170.72 (s) |
| C13/ C16 | 20.71 (q) | 20.61 (q) |

### 1.18 Preparation of (3-hydroxy-2,2,4,4-tetramethyl-cyclobutyl) acetate

| **Compound** | **MW** | **Mass/Volume** | **Moles** |
|---|---|---|---|
| 2,2,4,4-tetramethyl-cyclobutane-1,3-diol | 144.21 | 10 g | 0.069 |
| acetic anhydride | 102.09 | 7.08 g | 0.069 |
| N,N'-dimethylaminopyridine | 122.17 | 0.254 g | 0.002 |
| Tetrahydrofuran | | 50 mL | |

DMAP was added to the THF solution of the diol at RF. The mixture was set to 40° C and acetic anhydride (1eq) was slowly added at this temperature. After 3 h the reaction was cooled down to room temperature and slowly quenched with 50 mL of water, 50 mL of ethyl acetate. The organic phase was separated and washed with NaHCO₃ and brine solution. The organic extracts were combined and dried with sodium sulfate. After evaporating the solvent at reduced pressure, 7.2 g of crude product that contained 17.4% of the caged diol, 46.7% of the mono-acetate and 35.7% of the di-acetate according to the GC (area %). The product was purified by silica gel chromatography. The corresponding mono-acetate was obtained with a purity of 99% (GC area %) as a cis:trans isomer mixture in a ratio 1:1 (GC-analysis).

### 1.19 Preparation of (3-formyloxy-2,2,4,4-tetramethyl-cyclobutyl) acetate

| **Compound** | **MW** | **Mass/Volume** | **Moles** |
|---|---|---|---|
| mono-acetate of 2,2,4,4-tetramethyl-cyclobutane-1,3-diol | 186.25 | 7 g | 0.025 |
| formic acid | 46.03 | 7.98 g | 0.173 |
| acetic anhydride | 102.09 | 15.17 g | 0.149 |
| N,N'-dimethylaminopyridine | 122.17 | 0.061 g | 0.0005 |
| tetrahydrofuran | | 40 mL | |

At 0° C formic acid was slowly added to acetic anhydride. The mixture was stirred 2 h at 55° C. The mixture was then cooled down to 0° C and 20 mL THF were added, at this temperature a solution of the raw mono-acetate derivative (prepared as described before) in 20 mL THF was slowly added. After the addition DMAP was added to the mixture. The reaction was stirred at room temperature for 3.5 h. 50 mL toluene were added to the mixture and the organic phase was washed 3 times with 50 mL of water. The organic phase was dried with sodium sulfate and the solvent was removed in the rotatory evaporator to give 6.8 g of crude product that contained 19.12% of the mono-formate, 43% of the mono-acetate-mono-formate and 33% of the di-acetate according to the GC (area %). The crude product was purified by silica gel chromatography. The corresponding mono-acetate-mono-formate was isolated with a purity of 52% (GC area %) together with 28% of the mono-formate and 18% of the di-acetate according to GC (area %). All the compounds had a ca. 1:1 cis:trans ratio (according to GC area %).

### 1.20 Preparation of (3-methoxy-2,2,4,4-tetramethyl-cyclobutyl) acetate

| **Compound** | **MW** | **Mass/Volume** | **Moles** |
|---|---|---|---|
| mono-methyl ether of 2,2,4,4-tetramethyl-cyclobutane-1,3-diol | 158.24 | 7 g | 0.033 |
| acetic anhydride | 102.09 | 3.387 g | 0.033 |
| N,N'-dimethylaminopyridine | 122.17 | 0.244 g | 0.002 |
| Tetrahydrofuran | | 50 mL | |

122 mg of DMAP were added to the THF solution of the mono-methyl ether derivative of 2,2,4,4-tetramethyl-cyclobutane-1,3-diol at RT. The mono-methyl ether derivative that is used as the starting material was prepared as described before, with 1.3 eq of Mel. The raw material obtained after this reaction had a ca. 50% content on the mono-methyl ether derivative and was used in this synthesis without further purification.

The mixture was set to 40° C and acetic anhydride (1eq) was slowly added at this temperature. After 5 h uncomplete conversion of the alcohol was observed and the 122 mg of DMAP were added to the mixture. The reaction was stirred at 40° C 4 h more. After this time, the reaction was cooled down to room temperature and slowly quenched with 50 mL of water, 50 mL of ethyl acetate. The organic phase was separated and washed with NaHCO₃ and brine solution. The organic extracts were combined and dried with sodium sulfate. After evaporating the solvent at reduced pressure, 5.6 g of crude product that contained 32% of the mixed ether-ester derivative was obtained, being the rest methyl ethers (21%) and the di-acetate (30%) according to the GC (area %). The product was purified by silica gel chromatography. The corresponding mono-methyl-ether-mono-acetate was obtained with a purity of 72% (GC area %) as a cis:trans isomer mixture in a ratio 1:1 being the rest the dimethyl-ether. The product distribution was also confirmed by NMR.

### 2. Olfactory tests

In order to test the quality and intensity of the odor of the compounds (I) of the present invention, scent strip tests were performed.

For this purpose, strips of absorbent paper were dipped into solution containing 1 to 10 % by weight solution of the compound (I) to be tested in ethanol. After evaporation of the solvent (about 30 sec.) the scent impression was olfactively evaluated by a trained perfumer.

The results of the scent test are summarized in table 1.

**Table 1: Results of the scent tests.**

| Compound | Description |
|---|---|
| 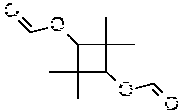 cis/trans: 55:45 | Woody, Sandel, Sweet, Amber, musk, Earthy, Camphor |
| 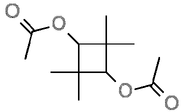 cis/trans: 55:45 | Woody (Sandel, cedar), pepper, tobacco, fruity, jasmin |
| 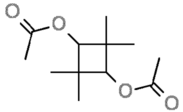 cis/trans: 9:1 | Woody (Sandel, cedar), pepper, tobacco, fruity, jasmin |
| 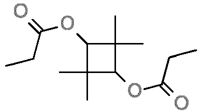 cis/trans: 1:1 | Spicy, (clove, pimento, cinnamon), woody, sweet, pine needle |
| 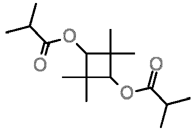 cis/trans: 55:45 | Yeasty, Fruity, pear, weak |
| 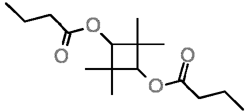 cis/trans: 55:45 | Yeasty, Garlic, weak |
| 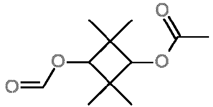 cis/trans: 1:1 | (Mixture comprising 52% of the mixed ester, 28% mono-formiate and 18% di-acetate) |
| | Woody, cedar, dried fruit (Damascone), Spicy, juniper, Sweet, candy ball |
| 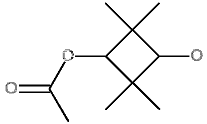 cis/trans: 1:1 | Weak, Herbal, Green |
| 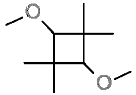 | Fresh, Marine, watery, medicinal, citrus, lemon, sweet |
| cis/trans: 4:1 | |
| 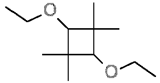 cis/trans: 3:1 | Fresh, Eukalyptus, Earthy, green, herbal, Juniper |
| 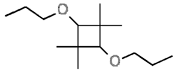 cis/trans: 85:15 | Fatty, weak |
| 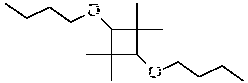 cis/trans: 85:15 | Banana, Dark cherry, Grapefruit, Sweet |
| 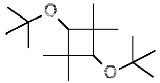 cis/trans: 2:1 | Cedarwood, Warm |
| 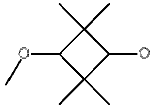 cis/trans: 6:94 | ozonic, ethereal, Herbal, clary sage |
| 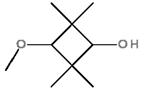 cis/trans: 98:2 | Sweet, Earthy, Pine needle |
| 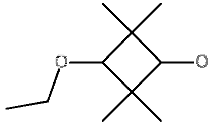 cis/trans: 55:45 | (Mixture of 80% monoethyl-ether and 20% diethyl ether) |
| | Weak, Woody, Pine, Pine needle, Natural |
| 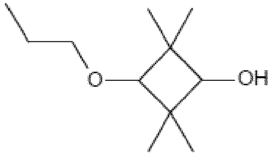 cis/trans: 2:3 | Chrysanthemum, Ethereal, Fresh |
| 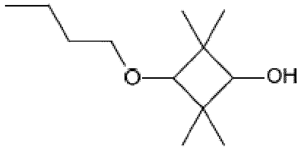 cis/trans: 60:40 | Sweet, Green beans, Aldehydic, Waxy |
| 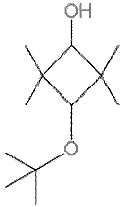 cis/trans: 2:3 | Sweet, Cedarwood, weak |
| 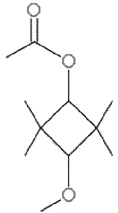 cis/trans: 50:50 | (Mixture of 72% mono-methyl-ether-mono-acetate and 28% dimethyl ether) |
| | Cedarwood, Terpenic Ambery, Pine needle |

## Claims

1. The use of a compound of the general formula (I) 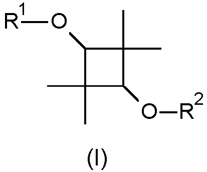 wherein
R¹ is C₁-C₄-alkyl or -(C=O)-R³,
R² is hydrogen, C₁-C₄-alkyl or -(C=O)-R⁴, and
R³ and R⁴, independently of one another, are selected from the group consisting of hydrogen and C₁-C₄-alkyl,
a stereoisomer thereof or a mixture of stereoisomers thereof,
as an aroma chemical.

2. The use according to claim 1, where in formula (I)
R¹ is C₁-C₄-alkyl and
R² is hydrogen or C₁-C₄-alkyl.

3. The use according to claim 2, where the compound of formula (I) has at least one of the following features a), b), c) and/or d)
a) R² is C₁-C₄-alkyl,
b) R¹ and R² are identical,
c) R¹ and R² are C₁-C₃-alkyl,
d) R¹ and R² are methyl or ethyl.

4. The use according to claim 1, where in formula (I)
R¹ is -(C=O)-R³ and
R² is hydrogen or -(C=O)-R⁴.

5. The use according to claim 4, where the compound of formula (I) has at least one of the following features e), f), g), h) and/or i)
e) R² is -(C=O)-R⁴,
f) R³ and R⁴ are identical,
g) R³ and R⁴ are selected from the group consisting of hydrogen and C₁-C₃-alkyl,
h) R³ and R⁴ are selected from the group consisting of hydrogen, methyl and ethyl,
i) R³ and R⁴ are methyl.

6. The use according to claim 1, where in formula (I)
R¹ is C₁-C₄-alkyl and
R² is -(C=O)-R³.

7. The use according to any of the preceding claims, in a composition selected from perfume compositions, cosmetic compositions, body care compositions, products for oral and dental hygiene, hygiene articles, cleaning compositions, textile detergent compositions, dishwashing compositions, compositions for scent dispensers, foods, food supplements, pharmaceutical compositions and crop protection compositions.

8. Aroma chemical composition comprising at least one compound of formula (I), a stereoisomer thereof or a mixture of stereoisomers thereof, according to any of claims 1 to 6, and at least one further compound (II) selected from the group consisting of further aroma chemicals different from compounds (I) and non-aroma chemical carriers;
where the composition is not a mixture comprising as compound of formula (I) 2,2,4,4-tetramethyl-cyclobutane-1,3-diol diacetate, and as only further compound(s) (II) acetic acid, optionally acetic anhydride and optionally zinc chloride;
where the composition is not a mixture comprising as compound of formula (I) 2,2,4,4-tetramethyl-cyclobutane-1,3-diol diformate, and as only further compounds (II) formic acid, benzene, optionally water, optionally sodium bicarbonate and optionally sodium sulfate;
where the composition is not a mixture comprising as compound of formula (I) 2,2,4,4-tetramethyl-cyclobutane-1,3-diol diisobutyrate, and as only further compounds (II) isobutyric acid, isobutyric anhydride and optionally zinc chloride;
where the composition is not a mixture comprising as compound of formula (I) 2,2,4,4-tetramethyl-cyclobutane-1,3-diol di-n-butyrate, and as only further compound(s) (II) n-butyric acid, optionally n-butyric anhydride and optionally zinc chloride;
where the composition is not a composition consisting of cyclobutane-1,3-diol diacetate and cellulose acetate butyrate;
where the composition is not a composition consisting of cyclobutane-1,3-diol diisobutyrate and cellulose acetate butyrate; and
where the composition is not a composition consisting of cyclobutane-1,3-diol din-butyrate and cellulose acetate butyrate.

9. The composition according to claim 8, selected from perfume compositions, cosmetic compositions, body care compositions, products for oral and dental hygiene, hygiene articles, cleaning compositions, textile detergent compositions, dishwashing compositions, compositions for scent dispensers, foods, food supplements, pharmaceutical compositions and crop protection compositions.

10. A method of preparing a fragranced ready-to-use composition, comprising incorporating at least one compound of formula (I), a stereoisomer thereof or a mixture of stereoisomers thereof, according to any of claims 1 to 6, into a ready-to-use composition;
where the ready-to-use composition is not a composition consisting of cyclobutane-1,3-diol diacetate and cellulose acetate butyrate;
where the ready-to-use composition is not a composition consisting of cyclobutane-1,3-diol diisobutyrate and cellulose acetate butyrate; and
where the ready-to-use composition is not a composition consisting of cyclobutane-1,3-diol di-n-butyrate and cellulose acetate butyrate.

11. The use of a compound of formula (I), a stereoisomer thereof or a mixture of stereoisomers thereof according to any of claims 1 to 6, for modifying the scent character of a fragranced ready-to-use composition.

12. A compound of the general formula (I.a) 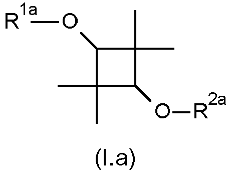 wherein
R^{1a} is C₂-C₄-alkyl and
R^{2a} is hydrogen or C₂-C₄-alkyl,
a stereoisomer thereof or a mixture of stereoisomers thereof;
where preferably the compound of formula (I.a) has at least one of the following features j), k) and/or l)
j) R^{1a} and R^{2a}, independently of one another, are selected from C₂-C₄-alkyl,
k) R^{1a} and R^{2a} are identical,
l) R^{1a} and R^{2a} are selected from the group consisting of ethyl, n-propyl and n-butyl.

13. A compound of the general formula (I.b) 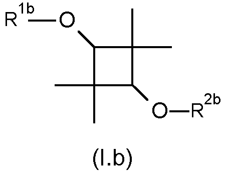 wherein
R^{1b} is -(C=O)-R³,
R^{2b} is hydrogen or -(C=O)-R⁴ and
R³ and R⁴, independently of one another, are selected from the group consisting of hydrogen and C₁-C₄-alkyl, with the provision that R³ and R⁴, if present, are different,
a stereoisomer thereof or a mixture of stereoisomers thereof;
except for 2,2,4,4-tetramethylcyclobutanediol monobutyrate;
where preferably the compound of formula (I.b) has at least one of the following features m), n) and/or o)
m) R^{2b} is -(C=O)-R⁴,
n) R³ and R⁴ are selected from the group consisting of hydrogen and C₁-C₃-alkyl,
o) R³ and R⁴ are selected from the group consisting of hydrogen, methyl and ethyl.

14. A method for preparing the compound of formula (I.a), a stereoisomer thereof or a mixture of stereoisomers thereof, according to claim 12, comprising
(i) reacting 2,2,4,4-tetramethylcyclobutane-1,3-diol with the alkylation reagent R^{1a}-X and optionally with the alkylation reagent R^{2a}-X, wherein R^{1a} and R^{2a} have one of the meanings given above and X represents a leaving group, selected from halogen, such as Cl, Br, I, and sulfonates, such as tosylate, mesylate, triflate or nonaflate, in the presence of a base to obtain a raw-product mixture, and
(ii) subjecting the raw-product mixture obtained in step (i) to a purification step.

15. A method for preparing the compound of formula (I.b), a stereoisomer thereof or a mixture of stereoisomers thereof, according to claim 13, comprising
(i) reacting 2,2,4,4-tetramethylcyclobutane-1,3-diol with the carboxylic acid R³-COOH or an anhydride thereof, or with the acid halogenide R³-(C=O)X' in the presence of an organic base and optionally with the carboxylic acid R⁴-COOH or an anhydride thereof, or with the acid halogenide R⁴-(C=O)X', wherein R³ and R⁴ have one of the meanings given above and X' represents a halogen, such as Cl, Br or I, to obtain a raw-product mixture, and
(ii) subjecting the raw-product mixture obtained in step (i) to a purification step.

## Patentansprüche

1. Verwendung einer Verbindung der allgemeinen Formel (I) 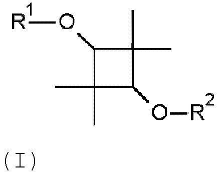 wobei
R¹ für C₁-C₄-Alkyl oder -(C=O)-R³ steht,
R² für Wasserstoff, C₁-C₄-Alkyl oder -(C=O)-R⁴ steht und
R³ und R⁴ unabhängig voneinander aus der Gruppe bestehend aus Wasserstoff und C₁-C₄-Alkyl ausgewählt sind;
eines Stereoisomers davon oder eines Gemischs von Stereoisomeren davon
als Aromachemikalie.

2. Verwendung nach Anspruch 1, wobei in Formel (I)
R¹ für C₁-C₄-Alkyl steht und
R² für Wasserstoff oder C₁-C₄-Alkyl steht.

3. Verwendung nach Anspruch 2, wobei die Verbindung der Formel (I) mindestens eines der folgenden Merkmale a), b), c) und/oder d) aufweist:
a) R² steht für C₁-C₄-Alkyl,
b) R¹ und R² sind gleich,
c) R¹ und R² stehen für C₁-C₃-Alkyl,
d) R¹ und R² stehen für Methyl oder Ethyl.

4. Verwendung nach Anspruch 1, wobei in Formel (I)
R¹ für -(C=O)-R³ steht und
R² für Wasserstoff oder -(C=O)-R⁴ steht.

5. Verwendung nach Anspruch 4, wobei die Verbindung der Formel (I) mindestens eines der folgenden Merkmale e), f), g), h) und/oder i) aufweist:
e) R² steht für -(C=O)-R⁴
f) R³ und R⁴ sind gleich,
g) R³ und R⁴ sind aus der Gruppe bestehend aus Wasserstoff und C₁-C₃-Alkyl ausgewählt,
h) R³ und R⁴ sind aus der Gruppe bestehend aus Wasserstoff, Methyl und Ethyl ausgewählt,
i) R³ und R⁴ stehen für Methyl.

6. Verwendung nach Anspruch 1, wobei in Formel (I)
R¹ für C₁-C₄-Alkyl steht und
R² für -(C=O)-R³ steht.

7. Verwendung nach einem der vorhergehenden Ansprüche in einer Zusammensetzung, die aus Parfümzusammensetzungen, Kosmetikzusammensetzungen, Körperpflegezusammensetzungen, Produkten für die Mund- oder Zahnhygiene, Hygieneartikeln, Reinigungszusammensetzungen, Textilwaschmittelzusammensetzungen, Geschirrspülmittelzusammensetzungen, Zusammensetzungen für Duftspender, Lebensmitteln, Nahrungsergänzungsmitteln, pharmazeutischen Zusammensetzungen und Pflanzenschutzzusammensetzungen ausgewählt ist.

8. Aromachemikalienzusammensetzung, umfassend mindestens eine Verbindung der Formel (I), ein Stereoisomer davon oder ein Gemisch von Stereoisomeren davon nach einem der Ansprüche 1 bis 6 und mindestens eine weitere Verbindung (II), die aus der Gruppe bestehend aus weiteren Aromachemikalien, die von den Verbindungen (I) verschieden sind, und Nicht-Aromachemikalien-Trägern ausgewählt sind;
wobei es sich bei der Zusammensetzung nicht um ein Gemisch handelt, das als Verbindung der Formel (I) 2,2,4,4-Tetramethylcyclobutan-1,3-dioldiacetat und als einzige weitere Verbindung(en) (II) Essigsäure, gegebenenfalls Essigsäureanhydrid und gegebenenfalls Zinkchlorid umfasst;
wobei es sich bei der Zusammensetzung nicht um ein Gemisch handelt, das als Verbindung der Formel (I) 2,2,4,4-Tetramethylcyclobutan-1,3-dioldiformiat und als einzige weitere Verbindung(en) (II) Ameisensäure, Benzol, gegebenenfalls Wasser, gegebenenfalls Natriumhydrogencarbonat und gegebenenfalls Natriumsulfat umfasst;
wobei es sich bei der Zusammensetzung nicht um ein Gemisch handelt, das als Verbindung der Formel (I) 2,2,4,4-Tetramethylcyclobutan-1,3-dioldiisobutyrat und als einzige weitere Verbindung(en) (II) Isobuttersäure, Isobuttersäureanhydrid und gegebenenfalls Zinkchlorid umfasst;
wobei es sich bei der Zusammensetzung nicht um ein Gemisch handelt, das als Verbindung der Formel (I) 2,2,4,4-Tetramethylcyclobutan-1,3-di-n-butyrat und als einzige weitere Verbindung(en) (II) n-Buttersäure, gegebenenfalls n-Buttersäureanhydrid und gegebenenfalls Zinkchlorid umfasst;
wobei es sich bei der Zusammensetzung nicht um eine Zusammensetzung handelt, die aus Cyclobutan-1,3-dioldiacetat und Celluloseacetatbutyrat besteht;
wobei es sich bei der Zusammensetzung nicht um eine Zusammensetzung handelt, die aus Cyclobutan-1,3-dioldiisobutyrat und Celluloseacetatbutyrat besteht; und wobei es sich bei der Zusammensetzung nicht um eine Zusammensetzung handelt, die aus Cyclobutan-1,3-dioldi-n-butyrat und Celluloseacetatbutyrat besteht.

9. Zusammensetzung nach Anspruch 8, die aus Parfümzusammensetzungen, Kosmetikzusammensetzungen, Körperpflegezusammensetzungen, Produkten für die Mund- oder Zahnhygiene, Hygieneartikeln, Reinigungszusammensetzungen, Textilwaschmittelzusammensetzungen, Geschirrspülmittelzusammensetzungen, Zusammensetzungen für Duftspender, Lebensmitteln, Nahrungsergänzungsmitteln, pharmazeutischen Zusammensetzungen und Pflanzenschutzzusammensetzungen ausgewählt ist.

10. Verfahren zur Herstellung einer duftstoffhaltigen gebrauchsfertigen Zusammensetzung, umfassend das Einarbeiten mindestens einer Verbindung der Formel (I), eines Stereoisomers davon oder eines Gemischs von Stereoisomeren davon nach einem der Ansprüche 1 bis 6 in eine gebrauchsfertige Zusammensetzung;
wobei es sich bei der gebrauchsfertigen Zusammensetzung nicht um eine Zusammensetzung handelt, die aus Cyclobutan-1,3-dioldiacetat und Celluloseacetatbutyrat besteht;
wobei es sich bei der gebrauchsfertigen Zusammensetzung nicht um eine Zusammensetzung handelt, die aus Cyclobutan-1,3-dioldiisobutyrat und Celluloseacetatbutyrat besteht; und
wobei es sich bei der gebrauchsfertigen Zusammensetzung nicht um eine Zusammensetzung handelt, die aus Cyclobutan-1,3-dioldi-n-butyrat und Celluloseacetatbutyrat besteht.

11. Verwendung einer Verbindung der Formel (I), eines Stereoisomers davon oder eines Gemischs von Stereoisomeren davon nach einem der Ansprüche 1 bis 6 zur Modifizierung des Duftcharakters einer duftstoffhaltigen gebrauchsfertigen Zusammensetzung.

12. Verbindung der allgemeinen Formel (I.a) 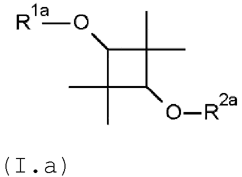 wobei
R^{1a} für C₂-C₄-Alkyl steht und
R^{2a} für Wasserstoff oder C₂-C₄-Alkyl steht,
ein Stereoisomer davon oder ein Gemisch von Stereoisomeren davon;
wobei vorzugsweise die Verbindung der Formel (I.a) mindestens eines der folgenden Merkmale j), k) und/oder l) aufweist:
j) R^{1a} und R^{2a} sind unabhängig voneinander aus C₂-C₄-Alkyl ausgewählt,
k) R^{1a} und R^{2a} sind gleich,
l) R^{1a} und R^{2a} sind aus der Gruppe bestehend aus Ethyl, n-Propyl und n-Butyl ausgewählt.

13. Verbindung der allgemeinen Formel (I.b) 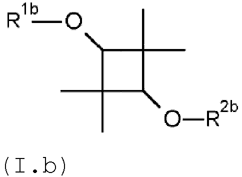 wobei
R^{1b} für -(C=O)-R³ steht,
R^{2b} für Wasserstoff oder -(C=O)-R⁴ steht und
R³ und R⁴ unabhängig voneinander aus der Gruppe bestehend aus Wasserstoff und C₁-C₄-Alkyl ausgewählt sind, mit der Maßgabe, dass R³ und R⁴, falls vorhanden, verschieden sind,
ein Stereoisomer davon oder ein Gemisch von Stereoisomeren davon;
mit Ausnahme von 2,2,4,4-Tetramethylcyclobutandiolmonobutyrat;
wobei vorzugsweise die Verbindung der Formel (I.b) mindestens eines der folgenden Merkmale m), n) und/oder o) aufweist:
m) R^{2b} steht für -(C=O)-R⁴,
n) R³ und R⁴ sind aus der Gruppe bestehend aus Wasserstoff und C₁-C₃-Alkyl ausgewählt,
o) R³ und R⁴ sind aus der Gruppe bestehend aus Wasserstoff, Methyl und Ethyl ausgewählt.

14. Verfahren zur Herstellung der Verbindung der Formel (I.a), eines Stereoisomers davon oder eines Gemischs von Stereoisomeren davon nach Anspruch 12, bei dem man:
(i) 2,2,4,4-Tetramethylcyclobutan-1,3-diol mit dem Alkylierungsreagens R^{1a}-X und gegebenenfalls mit dem Alkylierungsreagens R^{2a}-X, wobei R^{1a} und R^{2a} eine der oben angegebenen Bedeutungen haben und X für eine Abgangsgruppe, die aus Halogen, wie Cl, Br, I, und Sulfonaten, wie Tosylat, Mesylat, Triflat oder Nonaflat, ausgewählt ist, steht, in Gegenwart einer Base umsetzt, wobei man ein Rohproduktgemisch erhält, und
(ii) das in Schritt (i) erhaltene Rohproduktgemisch einem Reinigungsschritt unterwirft.

15. Verfahren zur Herstellung der Verbindung der Formel (I.b), eines Stereoisomers davon oder eines Gemischs von Stereoisomeren davon nach Anspruch 13, bei dem man:
(i) 2,2,4,4-Tetramethylcyclobutan-1,3-diol mit der Carbonsäure R³-COOH oder einem Anhydrid davon oder mit dem Säurehalogenid R³-(C=O)X' in Gegenwart einer organischen Base und gegebenenfalls mit der Carbonsäure R⁴-COOH oder einem Anhydrid davon oder mit dem Säurehalogenid R⁴-(C=O)X' umsetzt, wobei R³ und R⁴ eine der oben angegebenen Bedeutungen haben und X' für ein Halogen wie Cl, Br oder I stehen, wobei man ein Rohproduktgemisch erhält, und
(ii) das in Schritt (i) erhaltene Rohproduktgemisch einem Reinigungsschritt unterwirft.

## Revendications

1. Utilisation d'un composé de la formule générale (I) 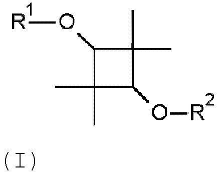 dans laquelle
R¹ est C₁-C₄-alkyle ou -(C=O)-R³,
R² est hydrogène, C₁-C₄-alkyle ou -(C=O)-R⁴, et
R³ et R⁴, indépendamment l'un de l'autre, sont choisis dans le groupe constitué par hydrogène et C₁-C₄-alkyle, d'un stéréoisomère correspondant ou d'un mélange de stéréoisomères correspondant,
comme composé chimique aromatique.

2. Utilisation selon la revendication 1, où dans la formule (I)
R¹ est C₁-C₄-alkyle et
R² est hydrogène ou C₁-C₄-alkyle.

3. Utilisation selon la revendication 2, où le composé de formule (I) possède au moins l'une des caractéristiques suivantes a), b), c) et/ou d)
a) R² est C₁-C₄-alkyle,
b) R¹ et R² sont identiques,
c) R¹ et R² sont C₁-C₃-alkyle,
d) R¹ et R² sont méthyle ou éthyle.

4. Utilisation selon la revendication 1, où dans la formule (I)
R¹ est -(C=O)-R³ et
R² est hydrogène ou -(C=O)-R⁴.

5. Utilisation selon la revendication 4, où le composé de formule (I) possède au moins l'une des caractéristiques suivantes e), f), g), h) et/ou i)
e) R² est -(C=O)-R⁴,
f) R³ et R⁴ sont identiques,
g) R³ et R⁴ sont choisis dans le groupe constitué par hydrogène et C₁-C₃-alkyle,
h) R³ et R⁴ sont choisis dans le groupe constitué par hydrogène, méthyle et éthyle,
i) R³ et R⁴ sont méthyle.

6. Utilisation selon la revendication 1, où dans la formule (I)
R¹ est C₁-C₄-alkyle et
R² est -(C=O)-R³.

7. Utilisation selon l'une quelconque des revendications précédentes, dans une composition choisie parmi les compositions de parfum, les compositions cosmétiques, les compositions de soins corporels, les produits d'hygiène bucco-dentaire, les articles d'hygiène, les compositions de nettoyage, les compositions de détergent pour textiles, les compositions pour lave-vaisselle, les compositions pour diffuseurs de parfum, les produits alimentaires, les compléments alimentaires, les compositions pharmaceutiques et les compositions de protection de cultures.

8. Composition chimique aromatique comprenant au moins un composé de formule (I), un stéréoisomère correspondant ou un mélange de stéréoisomères correspondant, selon l'une quelconque des revendications 1 à 6, et au moins un autre composé (II) choisi dans le groupe constitué par d'autres composés chimiques aromatiques différents des composés (I) et des supports chimiques non aromatiques ;
où la composition n'est pas un mélange comprenant comme composé de formule (I) le diacétate de 2,2,4,4-tétraméthyl-cyclobutane-1,3-diol et comme seul(s) autre(s) composé(s) (II) l'acide acétique, éventuellement l'anhydride acétique et éventuellement le chlorure de zinc ;
où la composition n'est pas un mélange comprenant comme composé de formule (I) le diformiate de 2,2,4,4-tétraméthyl-cyclobutane-1,3-diol et comme seul(s) autre(s) composé(s) (II) l'acide formique, le benzène, éventuellement l'eau, éventuellement le bicarbonate de sodium et éventuellement le sulfate de sodium ;
où la composition n'est pas un mélange comprenant comme composé de formule (I) du diisobutyrate de 2,2,4,4-tétraméthyl-cyclobutane-1,3-diol et comme seul(s) autre(s) composé(s) (II) l'acide isobutyrique, l'anhydride isobutyrique et éventuellement le chlorure de zinc ;
où la composition n'est pas un mélange comprenant comme composé de formule (I) le di-n-butyrate de 2,2,4,4-tétraméthyl-cyclobutane-1,3-diol, et comme seul(s) autre(s) composé(s) (II) l'acide n-butyrique, éventuellement l'anhydride n-butyrique et éventuellement le chlorure de zinc ;
où la composition n'est pas une composition constituée de diacétate de cyclobutane-1,3-diol et d'acétate butyrate de cellulose ;
où la composition n'est pas une composition constituée de diisobutyrate de cyclobutane-1,3-diol et d'acétate butyrate de cellulose ; et
où la composition n'est pas une composition constituée de di-n-butyrate de cyclobutane-1,3-diol et d'acétate butyrate de cellulose.

9. Composition selon la revendication 8, choisie dans le groupe constitué par des compositions de parfum, des compositions cosmétiques, des compositions de soins corporels, des produits d'hygiène bucco-dentaire, des articles d'hygiène, des compositions de nettoyage, des compositions de détergent pour textiles, des compositions pour lave-vaisselle, des compositions pour diffuseurs de parfum, des produits alimentaires, des compléments alimentaires, des compositions pharmaceutiques et des compositions de protection de cultures.

10. Procédé de préparation d'une composition fragrancée prête à l'emploi, comprenant l'incorporation d'au moins un composé de formule (I), d'un stéréoisomère correspondant ou d'un mélange de stéréoisomères correspondant, selon l'une quelconque des revendications 1 à 6, dans une composition prête à l'emploi ;
où la composition prête à l'emploi n'est pas une composition constituée de diacétate de cyclobutane-1,3-diol et d'acétate butyrate de cellulose ;
où la composition prête à l'emploi n'est pas une composition constituée de diisobutyrate de cyclobutane-1,3-diol et d'acétate butyrate de cellulose ; et
où la composition prête à l'emploi n'est pas une composition constituée de di-n-butyrate de cyclobutane-1,3-diol et d'acétate butyrate de cellulose.

11. Utilisation d'un composé de formule (I), d'un stéréoisomère correspondant ou d'un mélange de stéréoisomères correspondant selon l'une quelconque des revendications 1 à 6, pour modifier le caractère de parfum d'une composition fragrancée prête à l'emploi.

12. Composé de la formule générale (I.a) 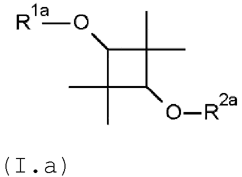 dans laquelle
R^{1a} est C₂-C₄-alkyle et
R^{2a} est hydrogène ou C₂-C₄-alkyle,
stéréoisomère correspondant ou mélange de stéréoisomères correspondant ;
où préférablement le composé de formule (I.a) possède au moins l'une des caractéristiques suivantes j), k) et/ou l)
j) R^{1a} et R^{2a}, indépendamment l'un de l'autre, sont choisis parmi C₂-C₄-alkyle,
k) R^{1a} et R^{2a} sont identiques,
l) R^{1a} et R^{2a} sont choisis dans le groupe constitué par éthyle, n-propyle et n-butyle.

13. Composé de la formule générale (I.b) 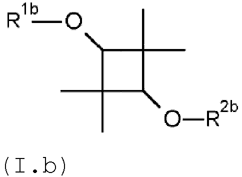 dans laquelle
R^{1b} est -(C=O)-R³,
R^{2b} est hydrogène ou -(C=O)-R⁴ et
R³ et R⁴, indépendamment l'un de l'autre, sont choisis dans le groupe constitué par hydrogène et C₁-C₄-alkyle, à la condition que R³ et R⁴, s'ils sont présents, soient différents,
stéréoisomère correspondant ou mélange de stéréoisomères correspondant ;
sauf pour le monobutyrate de 2,2,4,4-tetraméthylcyclobutanediol ;
où préférablement le composé de formule (I.b) possède au moins l'une des caractéristiques suivantes m), n) et/ou o)
m) R^{2b} est -(C=O)-R⁴,
n) R³ et R⁴ sont choisis dans le groupe constitué par hydrogène et C₁-C₃-alkyle,
o) R³ et R⁴ sont choisis dans le groupe constitué par hydrogène, méthyle et éthyle.

14. Procédé de préparation du composé de formule (I.a), d'un stéréoisomère correspondant ou d'un mélange de stéréoisomères correspondant, selon la revendication 12, comprenant
(i) la mise en réaction de 2,2,4,4-tétraméthylcyclobutane-1,3-diol avec le réactif d'alkylation R^{1a}-X et éventuellement avec le réactif d'alkylation R^{2a}-X, où R^{1a} et R^{2a} ont l'une des significations données ci-dessus et X représente un groupe partant, choisi parmi un halogène, tel que Cl, Br, I, et des sulfonates, tels que tosylate, mésylate, triflate ou nonaflate, en présence d'une base pour obtenir un mélange de produits bruts, et
(ii) la soumission du mélange de produits bruts obtenu à l'étape (i) à une étape de purification.

15. Procédé de préparation du composé de formule (I.b), d'un stéréoisomère correspondant ou d'un mélange de stéréoisomères correspondant, selon la revendication 13, comprenant
(i) la mise en réaction du 2,2,4,4-tétraméthylcyclobutane-1,3-diol avec l'acide carboxylique R³-COOH ou un anhydride correspondant, ou avec l'halogénure d'acide R³-(C=O)X' en présence d'une base organique et éventuellement avec l'acide carboxylique R⁴-COOH ou un anhydride correspondant, ou avec l'halogénure d'acide R⁴-(C=O)X', où R³ et R⁴ ont l'une des significations données ci-dessus et X' représente un halogène, tel que Cl, Br ou I, pour obtenir un mélange de produits bruts, et
(ii) la soumission du mélange de produits bruts obtenu à l'étape (i) à une étape de purification.
